# EUROPÄISCHE PATENTANMELDUNG

(11) **EP 1 586 650 A1**
(43) Veröffentlichungstag der Anmeldung: **19.10.2005**
(21) Anmeldenummer: 04009094.6
(22) Anmeldetag: 16.04.2004
(51) Int. Cl.: C12N 15/65, C12N 15/67, C12N 15/12, C12N 5/10, C07K 14/62

(54) **Eingriff in den Energiestoffwechsel von eukaryontenzellen zur Selektion und Expressionssteigerung**

(71) Anmelder: ProBioGen AG, 13086 Berlin (DE)
(72) Erfinder: Sandig, Volker, Dr., 13185 Berlin (DE); Jordan, Ingo, Dr., 10409 Berlin (DE); Winkler, Karsten, Dr., 10243 Berlin (DE)
(74) Vertreter: Helbing, Jörg

(57) **Zusammenfassung**

Die Erfindung betrifft ein Verfahren zur Kultivierung von Eukaryontenzellen, bei dem der Energiestoffwechsel der Zellen durch Einbringen von bestimmten Nukleinsäuresequenzen verbessert wird sowie geeignete transformierte Zellen hierfür und deren Verwendung. Die Nukleinsäuresequenzen kodieren für Energiestoffwechsel-relevante Faktoren wie Faktoren, die an der Nutzung von Zuckern und Kohlenhydraten beteiligt sind, wie z. B. das Glukosetransportprotein GLUT-1.

## Beschreibung

Die Erfindung betrifft ein Verfahren zur Kultivierung von Eukaryontenzellen, bei dem der Energiestoffwechsel der Zellen durch Einbringen von bestimmten Nukleinsäuresequenzen verbessert wird sowie geeignete transformierte Zellen hierfür und deren Verwendung. Die Nukleinsäuresequenzen kodieren für Energiestoffwechsel-relevante Faktoren wie Faktoren, die an der Nutzung von Zuckern und Kohlenhydraten beteiligt sind, wie z. B. das Glukosetransportprotein GLUT-1.

### Hintergrund der Erfindung

Wichtige Parameter für die Herstellung rekombinanter Wirkstoffe in Zellkulturen sind (1) der stabile Erhalt des Fremdgens, (2) eine hohe Expressionsrate des Fremdgens, und (3) Proliferationseigenschaften der transformierten Zelle. Die hier beschriebene Erfindung kann zur Optimierung aller drei Parameter beitragen.

In der Regel werden Fremdgene in Eukaryontenzellen über die Transfektion von Plasmiden exprimiert, die das entsprechende Gen unter der Kontrolle von cisaktiven Sequenzen wie Promotor und Polyadenylierungssignal tragen. Allerdings führt dieser Prozess nur zu einer vorübergehenden oder transienten Expression von Fremdgenen, da mit der Teilung der transfizierten Zellen und durch Degradation der eingebrachten DNA den Tochterzellen immer weniger Plasmide zur Verfügung stehen, so daß innerhalb weniger Passagen die transfizierten Plasmide nicht mehr nachweisbar sein können. Für eine stabile Expression muss das Fremdgen somit entweder in das Chromosom der Wirtszelle integriert, oder aber, vermittelt durch Funktionen im Plasmidrückrad, in seiner Replikation episomal an die Zellteilung gekoppelt werden (Van Craenenbroeck, K. et al., Eur. J. Biochem. 267:5665-78 (2000)).

Auch in der stabilen Konfiguration ist eine Expression in den allermeisten Fällen jedoch nicht dauerhaft: Episomen können im Laufe der Zellteilung ungleich auf Tochterzellen verteilt werden und so verloren gehen; chromosomale Abschnitte mit den integrierten Fremdgenen können durch Methylierung und Kondensierung für die zelluläre Transkriptionsmaschinerie unzugänglich gemacht werden oder durch Rekombination deletiert werden. Da das Einbringen von Fremdgenen häufig eine Belastung für die Wirtszelle bedeutet, sind die Zellen, in denen die Inaktivierung des Fremdgens erfolgt, gegenüber den stabilen Transfektanten im Vorteil. Im Laufe der Passagierung einer Zelllinie verdrängen die Revertanten somit ihre transgenen Nachbarn.

Dem Auftreten von Revertanten kann entgegengesteuert werden, indem die Expression des Fremdgens mit einer gleichzeitigen Expression eines Selektionsmarkers verbunden wird. Der Selektionsmarker kann ein Resistenzfaktor sein, der in der Lage ist, die Wirtszelle vor Antibiotika zu schützen, die für den Wildtyp toxisch sind.

Obwohl diese Form der Selektion sehr gute Ergebnisse liefert, sind einige erhebliche Nachteile zu berücksichtigen: (1) Nur teilweise inaktivierte Antibiotika, wie auch der Prozess der Inaktivierung, belasten die Zelle und können daher theoretisch mögliche Expressionsraten auf deutlich niedrigere Niveaus senken. (2) Vom Resistenzfaktor umgesetzte Antibiotika müssen erneuert werden. Dies erfordert Manipulationen an Kulturmedien, die zu Kontaminationen führen können. Die Abfolge von Verbrauch und Zufütterung führt außerdem zu zeitlichen Verläufen, in denen die Wirkstoffkonzentrationen zwischen unwirksamen und toxischen Werten schwanken können. (3) Das Resistenzgen ist in der Regel prokaryotischen Ursprungs. Von eukaryotischen Basenabfolgen im Gen und Primärsequenzmustern im Protein abweichende Abschnitte können von der Zelle als fremd erkannt werden und zur Inaktivierung des Fremdgens beitragen. In retroviralen Systemen wurde eine Inhibition der Expression durch die Neomycin-Phosphotransferase demonstriert (Artelt, P. et al., Gene 99:249-254 (1991)). In wieder anderen Systemen konnte eine Störung des RNA-Spleißens durch aminoglykosidische Antibiotika demonstriert werden (Waldsich, C. et al., RNA 4:1653-63 (1998)). (4) Antibiotika sind teuer und umweltbelastend. (5) In der Produktaufreinigung müssen Schritte zur Abtrennung und Inaktivierung der Antibiotika eingeplant werden (Panayotatos, N., Gene 74:357-63 (1988)).

Ein eukaryotisches Selektionssystem, das einige dieser Probleme umgeht, beruht auf der Expression rekombinanter Glutaminsynthetase (Barnes, L. M. et al., Cytotechnology 32:109-123 (2000)). Dieses Enzym katalysiert die Synthese von Glutamin durch Amidierung von Glutamat mit Ammoniumionen. Manche Zellinien haben dieses Enzym verloren und können somit nur in Medium überleben, das mit Glutamin supplementiert wurde. Für diese Zellen eignet sich eine Selektion in Medium ohne Glutaminzusatz, wobei das Fremdgen an die Expression der Glutaminsynthetase gekoppelt wird. Allerdings darf Glutamin dem Medium nur graduell entzogen werden, da bei einem zu schnellen Wechsel auf Glutaminfreies Medium auch die Zellen sterben, die rekombinante Glutaminsynthetase exprimieren.

Die Glutaminsynthetase kann außerdem als sogenannter amplifizierbarer Marker gezielt zur Herstellung von Hochexpressionszelllinien eingesetzt werden (Barnes, L. M. et al., Cytotechnology 32:109-123 (2000)). Diese Methode nutzt die Eigenschaft mancher Zellen, die Expressionsstärke einiger Genprodukte über Vervielfachung der Kopienzahl des zugehörigen Gens zu steigern. Für eine Genamplifikation der Glutaminsynthetase werden dem Medium graduell steigende Konzentration des spezifischen Inhibitors Methioninsulfoximin (MSX) zugegeben. Durch den Inhibitor sinkt die effektive Aktivität der Glutaminsynthetase. Dies wird durch Steigerung der Gendosis kompensiert. Dem Gen der Glutaminsynthetase eng benachbarte Fremdgene können in der so induzierten Genamplifikation ebenfalls vervielfältigt und damit verstärkt exprimiert werden. Die Genamplifikation kann auch dann erfolgreich sein, wenn die Zelle über eine endogene Glutaminsynthetase verfügt.

Einem vergleichbaren Prinzip folgt die Amplifikation über das Dihydrofolatreduktase (DHFR) Gen (Banerjee, D. et al., Cancer Gene Ther. 1:181-4 (1994)). Dieses Enzym überführt Folat über Dihydrofolat zu Tetrahydrofolat, einem essentiellen Edukt für die *de novo* Synthese von Purinen, Pyrimidinen und Glycin. DHFR wird durch Methotrexat (MTX) spezifisch inhibiert. Eine graduelle Zugabe von MTX kann eine Steigerung der Expression von DHFR über Amplifikation der Kopienzahl des Gens erzwingen. Wenn ein Fremdgen eng benachbart zu rekombinater DHFR in das Chromosom inseriert wird, dann kann durch die Zugabe von MTX mit der Amplifikation der rekombinanten DHFR auch eine Amplifikation der Zahl an Fremdgenkopien erreicht werden.

Auch wenn rekombinant in eine Zelle eingebrachte Genabschnitte im Chromosom stabilisiert und zu einer möglichst hohen Expression geführt worden sind, kann die Wirkstoffgewinnung im Bioreaktor scheitern, wenn die Kulturbedingungen zur Induktion von Apoptose führen. Dieses Problem ist in der Literatur bekannt (Frahm, B. et al., Bioprocess Biosyst. Eng. 26:1-10 (2003)) und wird durch Interferenz mit dem apoptotischen Programm minimiert. Wichtige Sensoren für Nährstoffmangel sind Proteine der Bcl-2-Genfamilie, wobei einige dieser Proteine pro-apoptotisch wirken und andere, wie das prototypische Bcl-2 Protein, eine begonnene apoptotische Signalkaskade stoppen können (Gross, A. et al., Genes Dev. 13:1899-911 (1999)). Rekombinante Überproduktion von Bcl-2 hat sich in der Wirkstoffherstellung als günstig erwiesen (Frahm, B. et al., Bioprocess Biosyst. Eng. 26:1-10 (2003)).

Es bestand jedoch weiterhin Bedarf für ein Selektionssystem, bei dem der Erhalt und die Hochexpression von Fremdgenen unabhängig von externen Selektionsmarkern möglich ist und gleichzeitig Induktion von Apoptose verhindert wird, also nicht - wie dies bei Bcl-2-Überproduktion der Fall wäre - der Abbruch eines schon laufenden Programms in einer vorgeschädigten Zelle erfolgt.

### Kurzbeschreibung der Erfindung

Es wurde nun gefunden, dass mittels einer bestimmten Manipulation - nämlich der Expression von Faktoren, die den Energiestoffwechsel der Zellen verbessern, insbesondere eine verbesserte Nutzung von Zuckern trotz gezielt oder verfahrenstechnisch bedingter Depletion von Nährstoffen oder Kofaktoren im Medium erlauben - besonders geeignete Zellen für die stabile Expression von Fremdgenen generiert werden können. In einer bevorzugten Variation der Erfindung können Zellkulturen unter widrigen Wachstumsbedingungen, zum Beispiel bei sehr hoher Zelldichte in Bioreaktoren oder bei Implantation als Zellkapsel in einen Organismus, vor Nekrose und/oder Induktion von Apoptose geschützt werden.

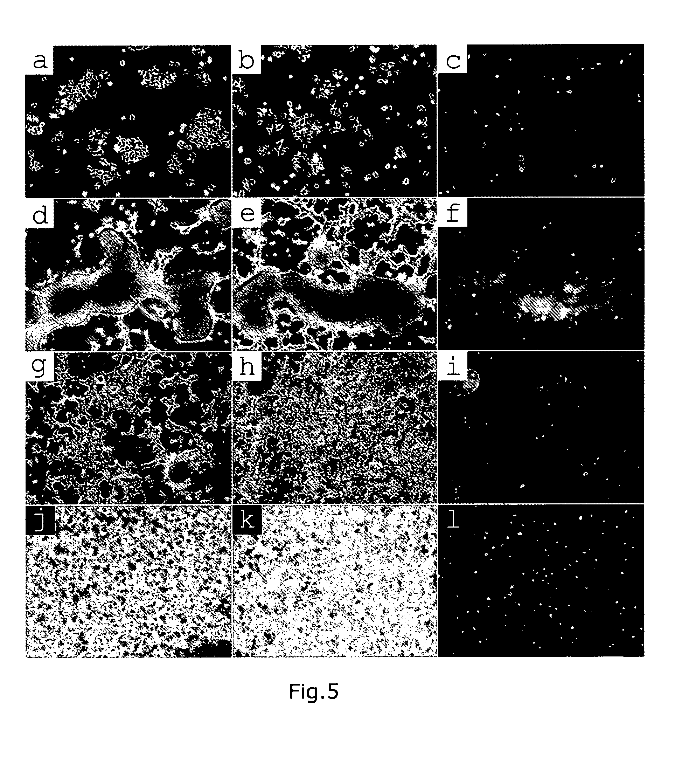

Einige Zellinien, wie zum Beispiel CHO Zellen, reagieren auf die vorstehend skizzierten Mangelbedingungen mit Apoptose, andere, wie zum Beispiel HEK 293 Zellen, verlangsamen die Proliferation (Zhangi, J.A. et al., Biotechnol. Bioeng. 64:108-119 (1999) und eigenen Beobachtungen des Anmelders). Die 293 Zellen werden im Zusammenhang mit Fig. 5 kurz diskutiert. Zellen, die nun jedoch den rekombinanten Faktor exprimieren sind besser befähigt, die niedrige Nährstoffkonzentration im Kulturüberstand zu nutzen und können proliferieren.

Überraschend ist hierbei außerdem, dass sich die Kultur durch den Verbrauch von Zuckern sukzessive selbst die Bedingungen einstellt, die zu einem verstärkten Selektionsdruck hin zu hochexprimierenden Zellen führen. Darüberhinaus ist es nicht erforderlich, das Nährmedium mit Antibiotika wie Gentamycin oder Hygromycin B zu versetzen, um den Erhalt von Fremdgenen zu gewährleisten.

Überraschenderweise wurde bei der Entwicklung des Systems beobachtet, dass die Produktionsraten von Fremdprotein in Zellen unter z. B. Glukosemangelbedingungen erhöht waren. Da aber die Proliferationsrate mit dem Glukoseangebot korrelierte, war folglich die spezifische Produktion (Produktionsrate pro Zelle) unter Glukosemangel erhöht und das Verhältnis von sekretiertem Fremdprotein zu anderen Komponenten im Kulturüberstand zugunsten einer vorteilhaften Aufreinigung verschoben. Es wird angenommen, dass im frühen Stadium des Zellstresses durch Glukosemangel die umfangreiche Aktivierung von Stressfaktoren auch zu einer metabolischen Aktivierung der Zelle führt, und damit zu erhöhten Expressionsleistungen für das Fremdgen.Fortwährender Glukosemangel induziert jedoch den Unfolded Protein Response und ist ausserdem Ursache für fehlerhafte Prozessierung von Glykoproteinen im endoplasmatischen Retikulum und Golgi Apparat (Kaufman, R. J. et al., Nat Rev Mol Cell Biol 3:411-21 (2002)). Das hier getestete und beschriebene System inhibiert möglicherweise die Induktion der massiven Stressantwort und erlaubt auch bei herabgesetzten Glukosekonzentrationen die Synthese normal prozessierter Glykoproteine. Diese Eigenschaften stellen einen erheblichen Vorteil in der Produktion von rekombinanten Proteinen und pharmazeutischen Wirkstoffen dar.

Niedrige Glukosewerte interferieren auch mit der Replikation einiger Viren, zum Beispiel Adenoviren (Bardell, D., Microbios 20:139-44 (1977)), die als virale Vektoren oder für die Impfstoffproduktion eine wichtige medizinische Bedeutung besitzten. Darüber hinaus wird Glukose im Medium durch die erhöhte Glykolyse in den infizierten Zellen (Bardell, D., Microbios 20:139-44 (1977)) schneller erschöpft. Da Glukose in höheren Konzentrationen jedoch toxisch für die Wirtszelle ist (Acker, H. et al., Cancer Res 47:3504-8 (1987); Medina-Navarro, R. et al., Hum Exp Toxicol 23:101-5 (2004)), kann der erhöhte Glukosebedarf nur eingeschränkt durch erhöhte Zugabe von Glukose kompensiert werden. Das hier beschriebene System erleichtert die Nutzung von Zuckern auch bei herabgesetzten Konzentrationen im Kulturmedium und erlaubt so verbesserte Ausbeuten in der pharmazeutischen Produktion von Viren oder viralen Vektoren.

Der Schutz der Zellen im Hochdichtereaktor vor Apoptose und/oder Nekrose folgt einem der Selektion verwandten Prinzip. Hierbei sind es jedoch vor allem lokale Variationen in der Nährstoffkonzentration, die das vorliegende System abpuffern soll. Auf diese Weise wird die Kultur vor nekrotischen oder apoptotischen Foci in Bereichen geringerer Diffusion geschützt, wodurch Kulturdauer und Produktionsintervalle verlängert werden sowie der Anteil von Abbauprodukten verringert wird.

Die Erfindung betrifft somit:
(1) ein Verfahren zur Kultivierung von Eukaryontenzellen (nachfolgend kurz "Zellen") umfassend die Unterstützung des Energiestoffwechsels der Zellen durch das Einbringen eines funktionellen Nukleinsäuresegments (d.h. in die Ausgangszellen), wobei dieses für einen Faktor kodiert, der den Energiestoffwechsel der Zellen im Vergleich zu den Ausgangszellen verbessert (wenn er in den Zellen exprimiert wird);
(2) ein Verfahren zur Expression eines Fremdgens in einer Eukaryontenzelle umfassend das Kultivieren einer Eukaryontenzelle, wie in (1) definiert, und Isolation des Expressionsprodukts aus der Kultur;
(3) Eukaryontenzellen, die für ein wie in (1) und (2) definiertes Verfahren geeignet sind, und mit einem funktionellen Nukleinsäuresegment, wie in (1) definiert, transformiert oder transfiziert ist;
(4) Verfahren zur Isolation von Eukaryontenzellen, wie in (3) definiert umfassend das Transformieren oder Transfizieren von Ausgangszellen mit einem funktionellen Nukleinsäuresegemt, wie in (1) definiert, und Selektieren unter Depletion eines den Faktor entsprechenden Nährstoffs oder Kofaktors;
(5) Funktionelles Nukleinsäuresegment, wie in (1) definiert; und
(6) die Verwendung von Zellen, wie in (3) definiert, zur Expression von Fremdgenen und zur Vermehrung von Viren.

### Beschreibung der Figuren

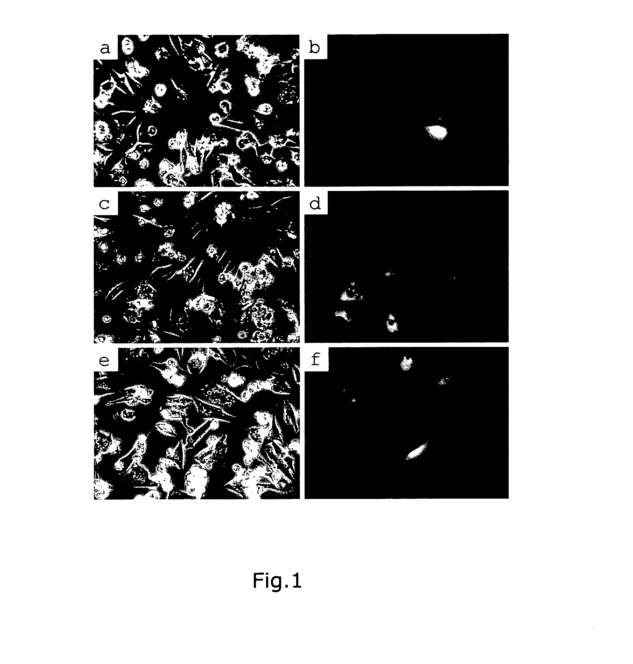

Fig. 1: CHO Zellen transfiziert mit pIJO-22-GlutGFP (SEQ ID NO:1). Gezeigt sind repräsentative Auschnitte der Kultur 7, 14 und 15 Tage nach Transfektion (jeweils a, b; c, d und e, f). Für jeden Zeitwert sind Phasenkontrastbilder (a, c, e) und Fluoreszenzaufnahmen zum Nachweis von GFP dargestellt (b, d, f). Induktion von Zelltod durch Glukosemangel wird ab Tag 14 deutlich. Die Dynamik dieses Prozesses wird deutlich, wenn die Zelldichte an den beiden aufeinanderfolgenden Tagen verglichen wird. Bevorzugt überleben Zellen, die die GLUT-GFP Kassette tragen.

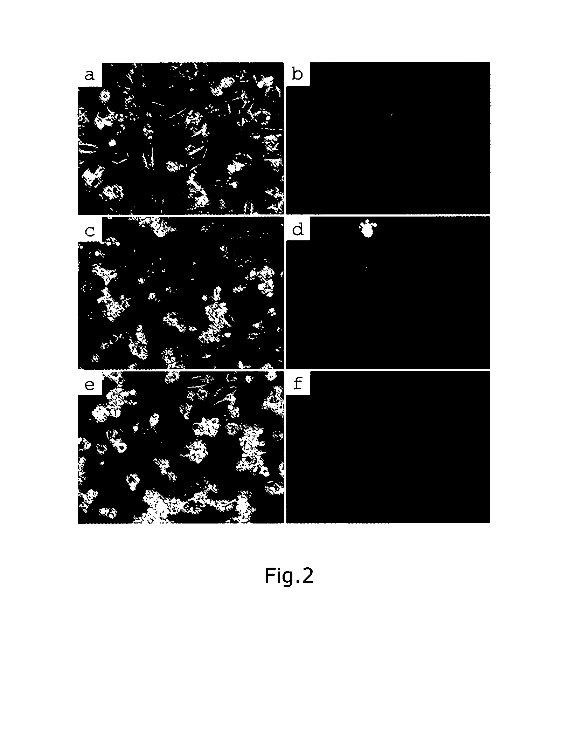

Fig. 2: Paralleles Kontrollexperiment zu Fig. 1: CHO Zellen transfiziert mit pIJO-25-NeoGFP (SEQ ID NO:2). Gezeigt sind repräsentative Auschnitte der Kultur 7, 14 und 15 Tage nach Transfektion (jeweils a, b; c, d und e, f). Für jeden Zeitwert sind Phasenkontrastbilder (a, c, e) und Fluoreszenzaufnahmen zum Nachweis von GFP dargestellt (b, d, f). Ab Tag 14 wird ein gleichförmiges Absterben der Kultur deutlich, unabhängig von der Gegenwart des transfizierten Episoms. Die Transfektionseffizienz für pIJO-22-Glut-GFP (SEQ ID NO:1) in Fig. 1 und für pIJO-25-NeoGFP (SEQ ID NO:2) in Fig. 2 war identisch.

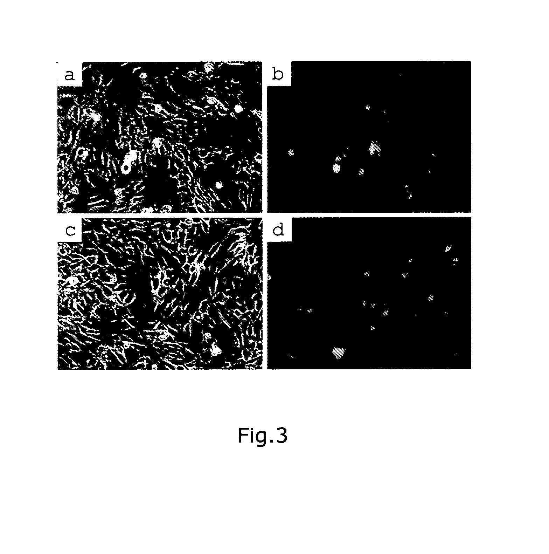

Fig. 3: Mit pIJO-22-GlutGFP (SEQ ID NO:1) (a, b) oder pIJO-25-NeoGFP (SEQ ID NO:2) Kontrollplasmid (c, d) transfizierte CHO Zellen wurden mit Hygromycin B vorselektioniert und ohne Mediumwechsel solange kultiviert, bis die Kulturen auf etwa 5% Konfluenz abgestorben sind. Anschließend wurde die Kultur durch regelmäßige Medienwechsel bis auf Konfluenz kultiviert und im Phasenkontrast (a, c) und unter Fluoreszenzfilter zum Nachweis des Reporterproteins (b, d) photographiert. Durch die widrigen Kulturbedingungen konnten im vorselektionierten Pool Subpopulationen entstehen, die das Episom verloren haben. Dieser Effekt war deutlich ausgeprägt in der Transfektion mit dem Referenzplasmid (NeoGFP Kassette; SEQ ID NO:2), nicht aber mit dem Effektorplasmid (GlutGFP Kassette; SEQ ID NO:1)).

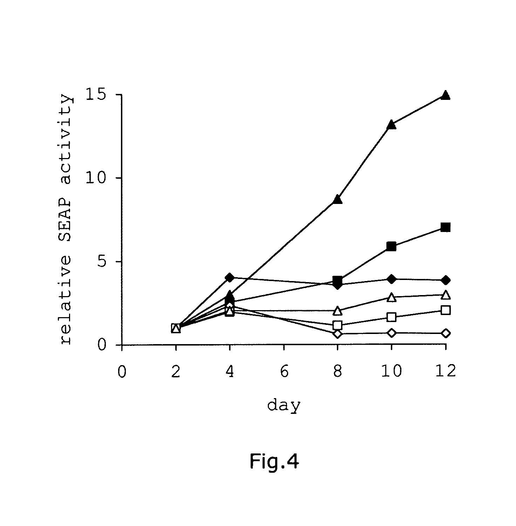

Fig. 4: Deutlicher Anstieg der Expression von selektierter alkalischer Phosphatase (SEAP) in Vektor pIJO-76 (GLUT-PV-SEAP) (SEQ ID NO:3) in Minimalmedium. Gegenüber der Referenz (pIJO-73 (PV-SEAP) (SEQ ID NO:4) in Minimalmedium) ist zum Endpunkt der Messung die SEAP-Aktivität 6fach höher. Auch in anderen Medien (Vollmedium oder Vollmedium mit Selektionsreagenz G418 auf 600 µg/ml) ist die Expression von pIJO-76 (GLUT-PV-SEAP) (SEQ ID NO:3) besser als beim Referenzplasmid pIJO 73 (PV-SEAP). Volle Symbole für Experimente mit Effektorplasmid pIJO-76 (GLUT-PV-SEAP) (SEQ ID NO:3), offene Symbole für Referenzplasmid pIJO 73 (PV-SEAP); Rauten für Kultivierung in Vollmedium, Quadrate für Vollmedium zusammen mit G418, Dreiecke für Minimalmedium.
Fig. 5: 293-Zellen mit Plasmid mit episomaler GLUT-IRES-GFP Kassette (pIJO-71 (SEQ ID NO:5)) wachsen in unsupplementiertem Medium und unter Glukosemangel besser als Wildtypzellen. Dargestellt sind Wildtypzellen in der linken Säule und transfizierte Zellen in den beiden rechten Säulen. Die transfizierten Zellen wurden vor Beginn dieses Experiments für 8 Wochen mit G418 selektioniert. Unsupplementiertes Glukose-Mangelmedium M4566) wurde für die Dauer des Experiments nicht gewechselt (Abbildungen a-i. Gleiche Anfangsbedingungen sind in a-c dokumentiert. Nach 4 Wochen Kultivierung (vergleiche g mit h und i), nicht aber nach 2 Wochen (d-f), wird der Vorteil für die Transfektanten deutlich. Als Referenz in Abbildungen j-l Zelldichten für Wildtyp und Transfektant in unsupplementiertem F10 Medium.

### Detaillierte Beschreibung der Erfindung

In dem Verfahren gemäß Ausführungsform (1) der Erfindung wird der Energiestoffwechsel der Zellen gegenüber den Ausgangszellen verbessert und in den Zellen exprimiert.

Das funktionelle Nukleinsäuresegment codiert vorzugsweise für einen Faktor, der die Nutzung von Mono-, Di- oder Oligosacchariden gegenüber den Ausgangszellen verbessert. Der unterstützte Energiestoffwechselweg ist dabei vorzugsweise ausgewählt aus Glykolyse, Citratzyklus, Pentosephosphatweg, Gluconeogenese, wobei der bevorzugte Energiestoffwechselweg die Glykolyse ist.

In dem erfindungsgemäßen Verfahren können als Ausgangszellen bzw. Zellen Vertebraten- und Invertebraten-Zellen mit Pflanzenzellen eingesetzt werden, wobei Säugerzellen (wie Nager-, Hund-, Schweine-, Rind- oder Primatenzellen (einschließlich humaner Zellen)) besonders bevorzugt sind. Ebenfalls sind Zellen von niederen Eukaryonten, wie Hefen und Spaltpilzen einsetzbar. Zellen im Sinne der vorliegenden Erfindung umfasst auch Gewebe/Gewebekulturen sowie (nichthumane) Organismen aus den vorstehend definierten Zellen. Es können in dem erfindungsgemäßen Verfahren primäre oder transformierte Zellen eingesetzt werden, wobei CHO-Zellen besonders bevorzugt sind.

In dem erfindungsgemäßen Verfahren (1) kann das funktionelle Nukleinsäuresegment transient oder stabil in die Zellen eingebracht werden. Das Nukleinsäuresegment kann ein DNA-Segment (Vektor, Plasmid u.s.w.) oder RNA-Segment (viraler RNA Vektor) sein. Die Kultivierung kann *in vitro* oder *in vivo* erfolgen, erfolgt jedoch vorzugsweise *in vitro.* Dies erflolgt durch - für auf diesem Gebiet tätigem Fachmann - geläufige Verfahren, wie Transformation und Transfektion mit geeigneten Konstrukten (Vektoren, Plasmiden, etc) umfassend das vorstehend definierte Nukleinsäuresegment. Das funktionelle Nukleinsäuresegment kann hierfür weitere funktionelle Sequenzen umfassen, vorzugsweise Promotoren, zu exprimierende Fremdgene, IRES-Elemente, Selektionsmarker, cis- und trans-aktive Faktoren zur verbesserten Translation einer mRNA oder für die Erkennung durch virale Faktoren z.B. zur Verpackung in Hüllen oder Replikation etc.

In einem weiteren erfindungsgemäßen Verfahren (1) kann der den Energiestoffwechsel beeinflussende Faktor z. B. ein Zucker-Transportprotein, vorzugsweise ein Pentose- oder Hexose-Transportprotein, besonders bevorzugt ein Glukose-Transportprotein sein, das eine Diffusion von Glukose in die Zellen erleichtert und/oder Glukose durch ein aktives Symportsystem in die Zelle transportiert. Besonders bevorzugt ist, dass der Faktor ein GLUT-1-, GLUT-2-, GLUT-3-, GLUT-4-, GLUT-5- oder GLUT-7-Protein ist.

In dem erfindungsgemäßen Verfahren (1) weist die Ausgangszelle wenigstens eine Kopie des den Faktor kodierenden Gens auf. In einer alternativen Ausführungsform ist das Gen in der Ausgangszellen inaktiviert. Das hier beschriebene System funktioniert auch in Zellen (CHO und HEK 293), die über intakte endogene Stoffwechselwege verfügten, die durch ebendas System komplementiert werden. In dem erfindungsgemäßen Verfahren (1) weist die Ausgangszelle wenigstens eine Kopie des den Faktor kodierenden Gens auf. In einer alternativen Ausführungsform ist das Gen in der Ausgangszelle inaktiviert. Der Einsatz von relevanten knock-out Mutanten würde den beobachteten Selektionseffekt verstärken. Eine knock-out Mutation des aktiven Transprortes SGLT-1 wurde duch niedrige Natriumkonzentration und Abwesenheit von Pyruvat als alternativen Energiedonor im Medium simuliert.

In einer besonders bevorzugten Ausführungsform des Verfahrens (1) ist die Expression des Zucker-Transportproteins mit der Expression eines Fremdgens verbunden. Vorzugsweise wird über Regulation der Menge an Zucker im Kulturmedium die Kultivierbarkeit, insbesondere Überleben und Proliferation der rekombinanten Zellpopulation gezielt unterstützt.

In einer anderen bevorzugten Ausführungsform des Verfahrens (1) umfasst das Verfahren eine Vorselektion mittels eines herkömmlichen Selektionsprinzips. Dies kann z. B. dadurch erfolgen, dass ein Inhibitor für den rekombinanten oder endogenen Transporter zur Selektion der Gen-Amplifikation zugegeben wird. Alternativ kann die Expression des Zucker-Transporters mit der Expression eines für ein Produkt kodierenden Fremdgens verbunden sein und die spezifische Ausbeute an Produkt durch verfahrensbedingte Nährstoffdepletion gesteigert werden, wobei die Zellen durch den rekombinanten Zucker-Transporter vor Apoptose, Nekrose oder Inhibition der Proliferation geschützt werden.

Gemäß dem erfindungsgemäßen Verfahren kann der Einsatz der Zellen im Bioreaktor, Gewebe oder Organismus derart gestaltet werden, dass verfahrensbedingte Nährstoffdepletion besser als durch die Ausgangszellen abgepuffert werden kann. Die transformierten Zellen, die mittels des erfindungsgemäßen Verfahren (1) erhalten werden sind als Wirt für die gezielte Vermehrung von Viren oder viralen Vektoren geeignet.

Die vorliegende Erfindung wird nachfolgend am Beispiel von Glukose erläutert, dies soll jedoch den Schutzbereich der Erfindung nicht einschränken.

Glukose kann als polares Molekül nicht frei durch die Plasmamebran diffundieren sondern gelangt rezeptorvermittelt über energieabhängige Wege in das Zellinnere. Das hier vorgestellte System moduliert den ersten und geschwindigkeitsbestimmenden Schritt in der Nutzung von Glukose als Energieträger, indem die Translokation in das Zellinnere durch spezielle, rekombinant exprimierte Proteine unterstützt wird. Dadurch können sehr niedrige äußere Glukosekonzentrationen genutzt werden, während Wildtypzellen ihre Teilungsrate drosseln oder sogar, wie zum Beispiel CHO Zellen, mit programmiertem Zelltod reagieren (Zanghi, J. A. et al., Biotechnol. Bioeng. 64:108-19 (1999)). Weitere Vorteile dieses Systems liegen darin, daß ein eukaryotisches Gen der gleichen Spezies in die Zelle eingebracht werden kann, im Gegensatz zu vielen heutigen Selektionsmarkern, die prokaryotischer Herkunft sind. Außerdem ist mit dem hier beschriebenen System die Zugabe von Wirkstoffen wie Antibiotika nicht notwendig.

Säugerzellen sind mit zwei mechanistisch unterschiedlichen Transportsystemen für Glukose ausgestattet, der erleichterten Diffusion und dem aktiven Symport:

Eine erleichterte Diffusion wird von spezifischen Rezeptoren der GLUT Proteinfamilie (GLUT1 bis GLUT5 und GLUT7) katalysiert und erlaubt den Influx von Hexosen entlang eines Konzentrationsgradienten (Brown, G. K., J. Inherit. Metab. Dis. 23:237-46 (2000)). Im Zytoplasma werden die Hexosen durch Phosphorylierung dem Gradienten entzogen, so daß es nicht zum Rückstau kommt. GLUT1 bis GLUT5 sind integrale Proteine der Plasmamebran, GLUT7 wird auf den intrazellulären Membranen des endoplasmatischen Retikulums exprimiert.

Aktive Transporter bringen Metabolite wie Aminosäuren und Zucker unter Nutzung von elektrochemischen Gradienten in das Zytoplasma. In dieser sehr heterogenen Superfamilie von Proteinen erfolgt der Glukosetransport durch SGLT1 (Wright, E. M. et al., J. Exp. Biol. 196:197-212 (1994)), das den Na⁺-Konzentrationsgradienten in einem sogenannten Symport für eine aktive Anreicherung der Glukose in der Zelle nutzt. Der treibende Na⁺ Gradient wiederum wird unter Hydrolyse von ATP im Antiport von Na⁺ im Lumen gegen K⁺ aus dem extrazellulären Raum aufrecht erhalten.

Das hier vorgestellte System bringt einen rekombinanten Glukosetransporter in zwei Anwendungsbereichen in eukaryotische Zellen: (i) die Expression des Transporters wird an die Expression eines Fremdgens gekoppelt und ermöglicht so einen Selektionsdruck, der den Erhalt des Fremdgens gewährleistet; oder (ii) der Transporter kann auch unabhängig von einem Fremdgen exprimiert werden, um die Kultivierbarkeit von Zellen oder die Produktausbeute unter widrigen Wachstumsbedingungen zu verbessern.

In einer bevorzugten Ausführung des Systems werden Fremdgen und Glukosetransporter auf einer mRNA über ein IRES-Element miteinander verbunden. Der Glukosetransporter kann ein aktiver Transporter sein, oder ein Faktor, der die Diffusion der Nährstoffe erleichtert. Das IRES-Element erlaubt die Translation bicistronischer mRNA Moleküle und ermöglicht so eine enge Koppelung der Expression zweier Gene. Das Plasmid stellt cis- und trans-aktive Faktoren für einen Erhalt als Episom zur Verfügung. Dadurch kann ohne Integration die stabile Expression des Fremdgens erreicht werden. Die Zellen werden anfänglich mit einem oder mehren Antibiotika selektioniert und nach dem Aufbau einer stabilen Zelllinie ohne die Zugabe von Antibiotika unter Glukosemangelbedingungen gehalten.

In Aspekten der oben genannten Ausführungen wird die Expression von Fremdgen und Glukosetransporter über unabhängige Expression von zwei Promotoren auf dem gleichen Plasmidrückrad oder über unabhängige Expression durch Kotransfektion mit verschiedenen Plasmiden gewährleistet. In weiteren Aspekten wird die gemeinsame Expression über die Bildung von chimären, nicht weiter prozessierten Proteinen, oder über Fusionsproteine erreicht, die kotranslational (zum Beispiel durch ribosomalen Leserasterwechsel) oder posttranslational (zum Beispiel durch Proteolyse) voneinander getrennt werden.

In weiteren Aspekten wird die transiente Expression von Proteinen oder die stabile Integration von Plasmiden oder Genabschnitten angestrebt. Episomale Plasmide sind dann nicht notwendig. In wieder anderen Aspekten ist das System Bestandteil eines stabilen oder transienten viralen Vektorsystems, zum Beispiel eines retroviralen oder alphaviralen Vektors.

In weiteren Aspekten wird zur Anreicherung transfizierter oder transduzierter Zellen frei von Antibiotikazugabe nur unter Glukosemangelbedingungen kultiviert. Die Selektion mit Antibiotika und Glukosemangel erfolgt gleichzeitig oder in verschiedenen Kombinationen und Zeitintervallen.

In weiteren Aspekten wird die Selektion oder der Erhalt von Fremdgenen durch die Anwendung von Stress unterstützt. Stressoren können zum Beispiel Inhibitoren mitochondrialer Prozesse oder Variationen in der Insulingabe sein. Außerdem wird die Nutzbarkeit von Nährstoffen durch das Einstellen der Ionenkonzentrationen im Kulturmedium (vor allem der am aktiven Symport beteiligten Ionen Na⁺ und K⁺) moduliert.

In weiteren Aspekten wird das Prinzip des Systems mit oder ohne der zusätzlichen Expression eines Transporters an späteren Punkten im Energiehaushalt der Zelle genutzt. So können zum Beispiel die Glukohexokinase im Zytoplasma oder die Aconitase in den Mitochondrien, dem Ort des Citratzyklus, in ihrer Expression durch rekombinante Methoden oder in ihrer Aktivität über chemische oder biologische Induktion moduliert werden.

In einem weiteren Aspekt wird der für Fruktose spezifische Transporter GLUT5 exprimiert und das System mit Fruktose als Energieträger verwendet. In wieder anderen Aspekten wird eine Kombination von Transportern, zum Beispiel GLUT1 mit GLUT5, GLUT1 mit dem im Endoplasmatischen Retikulum angesiedelten GLUT7, oder SGLT1 mit GLUT1, und/oder eine Kombination von Zuckermolekülen oder auch Zuckerderivaten eingesetzt. Bei der Kombination von Substanzen wird zum Beispiel Glukose zur Selektion von SGLT1-rekombinanten Zellen und Genistein (Vera, J.C. et al., J. Biol. Chem. 271:8719-8724 (1996)) zur Hemmung endogener GLUT1 Transporter verwendet.

In weiteren Aspekten werden Änderungen der Nährstoffkonzentration und/oder spezifische Inhibitoren des Transporters und/oder Substanzen mit Chromosomen-destabilisierenden Eigenschaften zur Genamplifikation eines Fremdgens eingesetzt, das in seiner Expression an die Expression des Transporters gekoppelt ist. In einer Ausführung dieses Aspektes dienen spezifische Wirkstoffe dazu, endogene Transportprozesse zu inhibieren, so daß der rekombinante Transporter verstärkt genutzt werden muß. In wieder anderen Aspekten werden Zellen verwendet, deren endogene Hexosetransportmoleküle experimentell inaktiviert werden, zum Beispiel durch antisense oder knock-out Technologie.

In einer weiteren Ausführung wird der Transporter oder eine Kombination von Transporter und Fremdgen mit der Absicht in Zellen exprimiert, diesen eine günstigere Kultivierbarkeit oder ein verbessertes Überleben unter widrigen Bedingungen zu ermöglichen. Diese Zellen könnten zum Beispiel unter Hochzelldichtebedingungen im Bioreaktor eingesetzt werden.

In einem Aspekt dieser Ausführung kann die mit dem Transporter ausgestattete Zelle für den Einsatz in einem lebenden Organismus, zum Beispiel in der *ex-vivo* Gentherapie, oder für die Implantation eines Zellverbunds oder einer Zellkapsel bestimmt sein. Durch die Expression des Transporters kann eine für die Anwendung günstigere Zelle auch an Orten geringerer Nährstoffkonzentration oder Diffusion geschaffen werden.

Die Erfindung wird anhand der nachfolgenden Beispiele näher erläutert, die jedoch den Schutzbereich der Erfindung nicht einschränken.

### Beispiele

### Beispiel 1: Aufbau des GLUT-1 Systems

Gesamt-RNA wurde mit saurem Phenol aus HeLa Zellen extrahiert und mit dT-16mer Primern durch die SuperScript II Reverse Transkriptase (Invitrogen, Carlsberg CA, USA) in cDNA umgeschrieben. Wegen überraschend geringer Ausbeute an PCR Amplifikationsprodukten wurde die cDNA mit Primern i104 (SEQ ID NO:10) und i105 (SEQ ID NO:11) voramplifiziert, und diese PCR Reaktion in einer weiteren PCR Reaktion mit den eigentlichen Klonierungsprimern i100 (SEQ ID NO:6) und i101 (SEQ ID NO:7) eingesetzt. Die Amplifikationen und Klonierungen sind anhand der (in drei Bereichen untereinander differierenden) GenBank Sequenzen #NM006516 und #XM002033 geplant worden. Für eine gerichtete Klonierung in den episomalen Vektor pREP4 (Invitrogen, Carlsbad CA, USA) enthalten die Primer i100 (SEQ ID NO:6) und i101 (SEQ ID NO:7) unikale Restriktasen-Zielsequenzen (Hind III und Xho I). Der Vektor pREP4 exprimiert außerdem den Resistenzfaktor gegen Hygromycin B.

Um den Erhalt transfizierter episomaler Plasmide verfolgen zu können, wurde eine auf der EMCV-IHRES basierende Expressionskassette für das EGFP Reporterprotein (Clontech, Palo Alto CA, USA) unmittelbar nach dem offenen Leseraster für GLUT1 inseriert, womit das Plasmid pIJO-22-GlutGFP (SEQ ID NO:1) entsteht.

### Beispiel 2: Einsatz des GLUT-1 Systems

A. Selektion: Als Kontrolle für eine erfolgreiche Anreicherung von pIJO-22-GlutGFP (SEQ ID NO:1) aufgrund der Expression von GLUT1 wurde der Leserahmen für GLUT1 durch das Gen für den eukaryotischen Selektionsmarker Neomycin-Phosphotransferase (Neo) ersetzt, indem pIJO-22 mit Hind III und Xho I geöffnet wurde und Neo als Stu I und BstB I Fragment aus pEGFP-N1 (Clontech) nach Behandlung mit Klenow DNA Polymerase mit T4 DNA Ligase eingefügt wurde, womit das Plasmid pIJO-25-NeoGFP (SEQ ID NO:2) entsteht. Von Neo ist dem Fachmann bekannt, daß es nicht toxisch ist und Differenzen in der Anreicherung von pIJO-22-GlutGFP (SEQ ID NO:1) und pIJO-25-NeoGFP (SEQ ID NO:2) sollten sich somit auf die Gegenwart von GLUT1 rückführen lassen.
   Die Plasmide pIJO-22-GlutGFP (SEQ ID NO:1) und pIJO-25-NeoGFP (SEQ ID NO:2) wurden als liposomaler Komplex in CHO Zellen transfiziert und die Expression des GFP Markerproteins über 15 Tage hinweg verfolgt. Die Transfektion erfolgte in diesem und in weiteren Experimenten mit LipofectAMINE Plus (Invitrogen): CHO Zellen wurden in 12-Lochplatten am Tag vor der Transfektion ausgesäät. Für die Transfektion wurden 750 ng Plasmid DNA mit 50 µl Opti-MEM (Invitrogen) und 5 µl Plus Reagent gemischt, bei Raumteperatur inkubiert und nach 15 min mit 2 µl LipofectAMINE in 50 µl Opti-MEM versetzt. Nach weiteren 15 min wurde das Kulturmedium durch frische 400 µl Opti-MEM, vermischt mit dem Transfektionsmix, ersetzt. Nach 3 Stunden wurde 1 ml Medium zugegeben.
   Hierbei zeigte sich in anfänglichen Experimenten, dass das für liposomale Transfektionen empfohlene Serum-freie Opti-MEM zu viel Glukose enthält, so daß ein Selektionsdruck aufgrund der Umsetzung von Glukose nur langsam einsetzt. In weiteren Experimenten wurde daher das Transfektionsprotokoll für eine Transfektion in Glukose-reduziertem Medium eingestellt. In den hier beschriebenen Versuchen war Glukose-reduziertes Medium (Sigma Taufkirchen, D) MEM Medium #M4655 (Sigma Taufkirchen, D), das mit 5% Kälberserum supplementiert wurde. MEM #M4655 zeichnet sich durch relativ niedrige Glukose (1 g/L) und NaCl (6.8 g/l) Konzentrationen aus; der neuartigen Natur des vorliegenden Systems entsprechend wurde kein Medium mit noch geringeren Glukosekonzentrationen gefunden. Pyruvat ist diesem Medium nicht zugesetzt. Pyruvat ist das sehr energiereiche Produkt der Glykolyse und wird im anschließenden Zitratzyklus endgültig in Energie umgewandelt. Durch den Entzug von Pyruvat sind die Zellen gezwungen, Glukose zur Energiegewinnung in die Zelle zu transportieren.
   Fig. 1 und 2 zeigen den zeitlichen Verlauf der GFP Expression in CHO-Kulturen, die parallel mit pIJO-22-GlutGFP (SEQ ID NO:1) (Fig. 1) oder dem Kontrollplasmid pIJO-25-NeoGFP (SEQ ID NO:2) (Fig. 2) transfiziert wurden. Kultivierung beider Ansätze erfolgte in MEM #M4655. Nach anfänglich vergleichbarer Zahl GFP-exprimierender Zellen und vergleichbaren Signalintensitäten des Reporterproteins für beide Konstrukte kommt es nur in der mit pIJO-22-GlutGFP (SEQ ID NO:1) transfizierten Kultur zu einer deutlichen Anreicherung GFP-positiver Zellen. Außerdem zeigt sich, dass die GLUT-GFPexprimierenden Zellen auffallend weniger Zelltod im Mangelmedium erleiden als die Neo-GFP-Kontrollen.
B. Erhalt des Episomes unter Stress: Für Abbildung Fig. 3 wurden mit pIJO-22-GlutGFP (SEQ ID NO:1) und pIJO-25-NeoGFP (SEQ ID NO:2) transfizierte CHO Zellen durch herkömmliche Selektion mit 200 µg/ml Hygromycin B für 20 Tage angereichtert, 6-fach verdünnt in T25 Kulturflaschen unter MEM #M4655 überführt und anschliessend für 20 Tage ohne Mediumwechsel kultiviert.
   Diese äußerst widrigen Kulturbedingungen führten zu massiver Abnahme lebensfähiger Zellen auf etwa 5% Konfluenz und könnten den limitierenden Bedingungen in einem Reaktor ähnlich sein.
   Nach dieser Behandlung erfolgten bis zum Erreichen der Konfluenz (weitere 14 Tage) regelmäßige Medienwechsel gegen MEM #M4655 ohne Hygromycin. Abbildung Fig. 3 zeigt eine deutliche Anreicherung der GlutGFP-positiven Zellen im Vergleich zu den NeoGFP-exprimierenden Zellen.
C. Quantifizierung: Das GFP-Reporterprotein erlaubte eine überraschend deutliche Bestätigung der erfindungsgemäßen Funktion des Systems. Im folgenden sollen Expressionsergebnisse für einen anderen Reporter, die Sekretierte Alkalische Phosphatase (SEAP), quantifiziert werden. Dazu wurde in einem episomalen Vektor dem ORF für Glut eine Expressionskassette für SEAP nachgeschaltet. Da für die Sekretion der SEAP die ursprünglichen, aminoterminalen Sequenzen notwendig sein können, wurde als IRES-Element das des Poliovirus (PV) verwendet, welches sich von der EMCV-IRES darin unterscheidet, dass kein ATG-Initiatorkodon durch die IRES gestellt werden muss. Der episomale Vektor mit der Glut-PV IRES-SEAP Kassette wurde pIJO-76 (SEQ ID NO:3) genannt, und entstand durch Insertion des Glut-Gens aus pIJO-62 (SEQ ID NO:12) als 1602 bp-grosses Hind III Fragment in den Vektor pIJO-73 (SEQ ID NO:4). pIJO-62 ist ein Zwischenklon, der ein PCR Amplifikationsprodukt (mit Primern i100 (SEQ ID NO:6) und i101 (SEQ ID NO:7)) des Glut-Gens enthält; pIJO-73 (SEQ ID NO:4) ist ein EBV-episomaler Vektor, der eine PV-IRES-SEAP Kassette nach einer Multiple Cloning Site enthält; diese Kassette wurde durch Insertion von SEAP als Hind III (Klenow-behandelt) Not I Fragment aus pIJO-53 (SEQ ID NO:13) in mit Sma I und Not I geöffnetem Vektor pBS Polio erzeugt. Als Negativkontrolle dient in den Experimenten mit SEAP der ursprüngliche episomale Vektor (pIJO-73 (SEQ ID NO:4)), dem der ORF für Glut ersatzlos fehlt. Der für die SEAP-Experimente verwendete episomale Vektor trägt das Resistenzgen für die Inaktivierung von G418 (Neo, Neomycinphosphotransferase).
   CHO Zellen wurden mit pIJO-76 (GLUT-PV-SEAP) (SEQ ID NO:3) und pIJO-73 (PV-SEAP) (SEQ ID NO:4) transfiziert und SEAP Sekretion in das Medium über 2 Wochen hinweg verfolgt (Fig. 4). Durch die Normierung der Daten auf den jeweils ersten Wert (Tag 2) werden unterschiedliche Expressionsstärken zwischen pIJO-76 (GLUT-PV-SEAP) (SEQ ID NO:3) und pIJO-73 (PV-SEAP) (SEQ ID NO:4) ausgeglichen: die Expression von SEAP ist höher im Kontrollvektor pIJO-73 (PV-SEAP) (SEQ ID NO:4) als in pIJO-76 (GLUT-PV-SEAP), da der SEAP im Referenzplasmid kein Gen vorgelagert ist. Am Tag 12 beträgt der Hintergrund-bereinigte, absolute SEAP Wert des abgebildeten Versuchs 667889 relative Lichteinheiten für pIJO 76 (GLUT-PV-SEAP) in Minimalmedium, 546866 relative Lichteinheiten für pIJO 73 (PV-SEAP) in Vollmedium/G418 und 344'356 für pIJO 73 (PV-SEAP) in Minimalmedium.
   Ein weiterer, unerwarteter günstiger Effekt für das vorliegende System liegt darin, dass die Zelldichte für Kulturen in Minimalmedium deutlich unter der Dichte der Zellen im Vollmedium am gleichen Tag liegt, obwohl sich die Expressionsstärke der Gene nicht wesentlich unterscheidet. Damit ergibt sich eine höhere spezifische Expressionsaktivität der einzelnen Zelle im Minimalmedium. Dieser Effekt hat einen erheblichen Vorteil für die Produktion, da sich für die Reinigung des Produkts aus dem Kulturüberstand die Abreicherung von sekretierten Metaboliten einfacher gestaltet.
D. 293 Zellen: HEK 293 Zellen zeigten sich überraschenderweise unempfindlich gegenüber Glukoselimitierung. Trotz dieser für eine Selektion ungünstigen Vorbedingungen wurde das Glut-System auch in HEK 293 Zellen eingesetzt und erbrachte einen unvorhergesehenen Effekt: HEK 293 Zellen wurden mit pIJO-71 (EBN-GLUT-GFP) (SEQ ID NO:5) transfiziert und mit G418 für 8 Wochen selektioniert. Die stabilen Transfektanten wurden parallel mit wildtypischen HEK 293 als Referenz auf gleiche Konfluenz in verschiedenen Medien (ohne G418) vorgelegt: unsupplementiertes #M4655, unsupplementiertes F10 Medium, und Serum-supplementiertes PBG 1.0 Medium. Das Kulturmedium wurde für die folgenden 4 Wochen nicht gewechselt oder mit frischem Medium versetzt. Fig. 5 zeigt den Ausgangspunkt und zwei Momentaufnahmen (nach 2 und 4 Wochen). Überaschenderweise wurde nur unter Glukosemangel nach 4 Wochen, nicht aber nach 2 Wochen, ein deutlicher Unterschied in der Dichte der Zellen beobachtet. (F10 Medium enthält mit 1100 mg/l ebenfalls wenig Glukose, was jedoch durch 110 mg/l Natriumpyruvat kompensiert wird). Die Fluoreszenzaufnahme zeigt, dass das episomale Transgen ohne weitere G418-Selektion über die 4 Wochen Versuchsdauer vollständig erhalten worden ist. Die vorliegende Erfindung eignet sich somit unerwarteterweise auch zur Unterstützung der Langzeitkultivierung von Zelllinien, die auf Glukosemangel scheinbar nicht reagieren.

### Beispiel 3: Einrichtung des SGLT-1 Systems

Gesamt-RNA wurde mit saurem Phenol aus HeLa Zellen extrahiert und mit dT-16mer Primern durch die SuperScript II Reverse Transkriptase in cDNA umgeschrieben. Die Amplifikationen mit Primerpaar i102 (SEQ ID NO:8) und i103 (SEQ ID NO:9) sowie Klonierungen sind anhand der GenBank Sequenz #M24847 geplant worden. Für eine gerichtete Klonierung in den episomalen Vektor pREP4 enthalten die Primer i102 (SEQ ID NO:8) und i103 (SEQ ID NO:9) unikale Restriktasen-Zielsequenzen.

Nachdem anfängliche Amplifikationen über RT-PCR nicht erfolgreich waren, sind für weitere Versuche HeLa Zellen eingesetzt worden, die unter Glukosemangelbedingungen kultiviert waren. So sollte eine mögliche Induktion der Expression von SGLT1 erreicht werden, und damit eine relative Anreicherung der SGLT1 mRNA. Tatsächlich konnte aus derart behandelten Zellen isolierte RNA in der RT-PCR in ein überraschend breites Spektrum von Amplifikationsprodukten umgewandelt werden. Das Produktspektrum zeichnete sich durch sehr geringe Ausbeuten und einer Schar von Signalen aus, die auch im Bereich der erwarteten Größe von 2013 bp auf unterschiedlich große Produkte schließen lassen. Durch die Klonierung unterschiedlich großer Amplifikationsprodukte konnte die gewünschte Sequenz isoliert und in das episomale Plasmid pREP4 transferiert werden.

## Patentansprüche

1. Verfahren zur Kultivierung von Eukaryontenzellen (Zellen), umfassend die Unterstützung des Energiestoffwechsels der Zellen durch Einbringen eines funktionellen Nukleinsäuresegments in die Ausgangszellen, wobei dieses für einen Faktor kodiert, der den Energiestoffwechsel der Zellen gegenüber den Ausgangszellen verbessert.

2. Verfahren nach Anspruch 1, wobei der Faktor die Nutzung von Mono-, Di- oder Oligosacchariden gegenüber den Ausgangszellen verbessert.

3. Verfahren nach Anspruch 1 oder 2, wobei
(i) der unterstützte Energiestoffwechselweg ausgewählt ist aus Glykolyse, Citratzyklus, Pentosephosphatweg und Gluconeogenese und vorzugsweise Glykolyse ist; und/oder
(ii) die Zellen Vertebraten- oder Invertebraten-Zellen sind, vorzugsweise Säugerzellen sind; und/oder
(iii) die Zellen primäre oder transformierte Zellen und insbesondere CHO-Zellen sind; und/oder
(iv) das funktionelle Nukleinsäuresegment transient oder stabil in die Zellen eingebracht ist; und/oder
(v) das Nukleinsäuresegment ein DNA- oder RNA-Segment ist; und/oder
(vi) die Kultivierung *in vitro* oder *in vivo,* vorzugsweise *in vitro*, erfolgt.

4. Verfahren nach Anspruch 1, 2 oder 3, wobei
(i) der den Energiestoffwechsel beeinflussende Faktor ein Zucker-Transportprotein, vorzugsweise ein Hexose-Transportprotein, besonders bevorzugt ein Glukose-Transportprotein ist, das eine Diffusion von Glukose in die Zellen erleichtert und/oder Glukose durch ein aktives Symportsystem in die Zelle transportiert, besonders bevorzugt der Faktor ein GLUT-1-, GLUT-2-, GLUT-3-, GLUT-4-, GLUT-5- oder GLUT-7-Protein ist; und/oder
(ii) die Ausgangszellen wenigstens eine Kopie des den Faktor kodierenden Gens aufweist oder das Gen in der Ausgangszellen inaktiviert ist; und/oder
(iii) das funktionelle Nukleinsäuresegment weitere funktionelle Sequenzen, vorzugsweise Promotoren, zu exprimierende Fremdgene, IRES-Elemente, Selektionsmarker, cis- und trans-aktive Faktoren, zur verbesserten Translation einer mRNA oder für die Erkennung durch virale Faktoren, insbesondere zur Verpackung in Hüllen oder Replikation etc. aufweist.

5. Verfahren nach Anspruch 4, wobei
(i) die Expression des Zucker-Transportproteins mit der Expression eines Fremdgens verbunden ist und vorzugsweise über Regulation der Menge an Zucker im Kulturmedium die Kultivierbarkeit, insbesondere Überleben und Proliferation der rekombinanten Zellpopulation gezielt unterstützt wird; und/oder
(ii) eine Vorselektion mit einem herkömmlichen Selektionsprinzip stattfindet; und/oder
(iii) ein Inhibitor für den rekombinanten oder endogenen Transporter zur Selektion der Gen-Amplifikation zugegeben wird; und/oder
(iv) die Expression des Zucker-Transporters mit der Expression eines für ein Produkt kodierenden Fremdgens verbunden ist und die spezifische Ausbeute an Produkt durch verfahrensbedingte Nährstoffdepletion gesteigert wird, wobei die Zellen durch den rekombinanten Zucker-Transporter vor Apoptose, Nekrose oder Inhibition der Proliferation geschützt werden; und/oder
(v) der Einsatz der Zellen im Bioreaktor, Gewebe oder Organismus derart gestaltet wird, dass verfahrensbedingte Nährstoffdepletion besser als durch die Ausgangszellen abgepuffert werden kann; und/oder
(vi) die transformierten Zellen als Wirt für die gezielte Vermehrung von Viren oder viralen Vektoren angepasst werden.

6. Verfahren zur Expression eines Fremdgens in einer Eukaryontenzelle, umfassend das Kultivieren einer Eukaryontenzelle, wie in Ansprüchen 1 bis 5 definiert, und Isolation des Expressionsprodukts aus der Kultur.

7. Eukaryontenzellen, die für ein Verfahren gemäß den Ansprüchen 1 bis 6 geeignet sind, und mit einem funktionellen Nukleinsäuresegment, wie in Ansprüchen 1 bis 5 definiert, transformiert oder transfiziert sind.

8. Verfahren zur Isolation von Eukaryontenzellen gemäß Anspruch 7 umfassend das Transformieren oder Transfizieren von Ausgangszellen mit einem funktionellen Nukleinsäuresegment wie in Ansprüchen 1 bis 5 definiert und Selektion unter Depletion eines den Faktor entsprechenden Nährstoffs oder Kofaktors.

9. Funktionelle Nukleinsäuresegmente, wie in Ansprüchen 1 bis 5 definiert.

10. Verwendung von Zellen, wie in Anspruch 7 definiert, zur Expression von Fremdgenen und zur Vermehrung von Viren.


SEQUENZPROTOKOLL

<110> ProBioGen AG

<120> Eingriff in den Energiestoffwechsel von Eukaryontenzellen zur
      Selektion und Expressionssteigerung

<130> 040640ep/JH/PCH

<140>
<141>

<160> 13

<170> PatentIn Ver. 2.1

<210> 1
<211> 12977
<212> DNA
<213> Künstliche Sequenz

<220>
<223> Beschreibung der künstlichen Sequenz:
      pIJO-22-GlutGFP

<400> 1
gccccgccgc cggacgaact aaacctgact acggcatctc tgccccttct tcgctggtac 60
gaggagcgct tttgttttgt attcggggca gtgcatgtaa tcccttcagt tggttggtac 120
aacttgccaa ctgggccctg ttccacatgt gacacggggg gggaccaaac acaaaggggt 180
tctctgactg tagttgacat ccttataaat ggatgtgcac atttgccaac actgagtggc 240
tttcatcctg gagcagactt tgcagtctgt ggactgcaac acaacattgc ctttatgtgt 300
aactcttggc tgaagctctt acaccaatgc tgggggacat gtacctccca ggggcccagg 360
aagactacgg gaggctacac caacgtcaat cagaggggcc tgtgtagcta ccgataagcg 420
gaccctcaag agggcattag caatagtgtt tataaggccc ccttgttaac cctaaacggg 480
tagcatatgc ttcccgggta gtagtatata ctatccagac taaccctaat tcaatagcat 540
atgttaccca acgggaagca tatgctatcg aattagggtt agtaaaaggg tcctaaggaa 600
cagcgatatc tcccacccca tgagctgtca cggttttatt tacatggggt caggattcca 660
cgagggtagt gaaccatttt agtcacaagg gcagtggctg aagatcaagg agcgggcagt 720
gaactctcct gaatcttcgc ctgcttcttc attctccttc gtttagctaa tagaataact 780
gctgagttgt gaacagtaag gtgtatgtga ggtgctcgaa aacaaggttt caggtgacgc 840
ccccagaata aaatttggac ggggggttca gtggtggcat tgtgctatga caccaatata 900
accctcacaa accccttggg caataaatac tagtgtagga atgaaacatt ctgaatatct 960
ttaacaatag aaatccatgg ggtggggaca agccgtaaag actggatgtc catctcacac 1020
gaatttatgg ctatgggcaa cacataatcc tagtgcaata tgatactggg gttattaaga 1080
tgtgtcccag gcagggacca agacaggtga accatgttgt tacactctat ttgtaacaag 1140
gggaaagaga gtggacgccg acagcagcgg actccactgg ttgtctctaa cacccccgaa 1200
aattaaacgg ggctccacgc caatggggcc cataaacaaa gacaagtggc cactcttttt 1260
tttgaaattg tggagtgggg gcacgcgtca gcccccacac gccgccctgc ggttttggac 1320
tgtaaaataa gggtgtaata acttggctga ttgtaacccc gctaaccact gcggtcaaac 1380
cacttgccca caaaaccact aatggcaccc cggggaatac ctgcataagt aggtgggcgg 1440
gccaagatag gggcgcgatt gctgcgatct ggaggacaaa ttacacacac ttgcgcctga 1500
gcgccaagca cagggttgtt ggtcctcata ttcacgaggt cgctgagagc acggtgggct 1560
aatgttgcca tgggtagcat atactaccca aatatctgga tagcatatgc tatcctaatc 1620
tatatctggg tagcataggc tatcctaatc tatatctggg tagcatatgc tatcctaatc 1680
tatatctggg tagtatatgc tatcctaatt tatatctggg tagcataggc tatcctaatc 1740
tatatctggg tagcatatgc tatcctaatc tatatctggg tagtatatgc tatcctaatc 1800
tgtatccggg tagcatatgc tatcctaata gagattaggg tagtatatgc tatcctaatt 1860
tatatctggg tagcatatac tacccaaata tctggatagc atatgctatc ctaatctata 1920
tctgggtagc atatgctatc ctaatctata tctgggtagc ataggctatc ctaatctata 1980
tctgggtagc atatgctatc ctaatctata tctgggtagt atatgctatc ctaatttata 2040
tctgggtagc ataggctatc ctaatctata tctgggtagc atatgctatc ctaatctata 2100

tctgggtagt atatgctatc ctaatctgta tccgggtagc atatgctatc ctcatgcata 2160
tacagtcagc atatgatacc cagtagtaga gtgggagtgc tatcctttgc atatgccgcc 2220
acctcccaag ggggcgtgaa ttttcgctgc ttgtcctttt cctgcatgct ggttgctccc 2280
attcttaggt gaatttaagg aggccaggct aaagccgtcg catgtctgat tgctcaccag 2340
gtaaatgtcg ctaatgtttt ccaacgcgag aaggtgttga gcgcggagct gagtgacgtg 2400
acaacatggg tatgcccaat tgccccatgt tgggaggacg aaaatggtga caagacagat 2460
ggccagaaat acaccaacag cacgcatgat gtctactggg gatttattct ttagtgcggg 2520
ggaatacacg gcttttaata cgattgaggg cgtctcctaa caagttacat cactcctgcc 2580
cttcctcacc ctcatctcca tcacctcctt catctccgtc atctccgtca tcaccctccg 2640
cggcagcccc ttccaccata ggtggaaacc agggaggcaa atctactcca tcgtcaaagc 2700
tgcacacagt caccctgata ttgcaggtag gagcgggctt tgtcataaca aggtccttaa 2760
tcgcatcctt caaaacctca gcaaatatat gagtttgtaa aaagaccatg aaataacaga 2820
caatggactc ccttagcggg ccaggttgtg ggccgggtcc aggggccatt ccaaagggga 2880
gacgactcaa tggtgtaaga cgacattgtg gaatagcaag ggcagttcct cgccttaggt 2940
tgtaaaggga ggtcttacta cctccatata cgaacacacc ggcgacccaa gttccttcgt 3000
cggtagtcct ttctacgtga ctcctagcca ggagagctct taaaccttct gcaatgttct 3060
caaatttcgg gttggaacct ccttgaccac gatgctttcc aaaccaccct ccttttttgc 3120
gcctgcctcc atcaccctga ccccggggtc cagtgcttgg gccttctcct gggtcatctg 3180
cggggccctg ctctatcgct cccgggggca cgtcaggctc accatctggg ccaccttctt 3240
ggtggtattc aaaataatcg gcttccccta cagggtggaa aaatggcctt ctacctggag 3300
ggggcctgcg cggtggagac ccggatgatg atgactgact actgggactc ctgggcctct 3360
tttctccacg tccacgacct ctccccctgg ctctttcacg acttcccccc ctggctcttt 3420
cacgtcctct accccggcgg cctccactac ctcctcgacc ccggcctcca ctacctcctc 3480
gaccccggcc tccactgcct cctcgacccc ggcctccacc tcctgctcct gcccctcctg 3540
ctcctgcccc tcctcctgct cctgcccctc ctgcccctcc tgctcctgcc cctcctgccc 3600
ctcctgctcc tgcccctcct gcccctcctg ctcctgcccc tcctgcccct cctcctgctc 3660
ctgcccctcc tgcccctcct cctgctcctg cccctcctgc ccctcctgct cctgcccctc 3720
ctgcccctcc tgctcctgcc cctcctgccc ctcctgctcc tgcccctcct gctcctgccc 3780
ctcctgctcc tgcccctcct gctcctgccc ctcctgcccc tcctgcccct cctcctgctc 3840
ctgcccctcc tgctcctgcc cctcctgccc ctcctgcccc tcctgctcct gcccctcctc 3900
ctgctcctgc ccctcctgcc cctcctgccc ctcctcctgc tcctgcccct cctgcccctc 3960
ctcctgctcc tgcccctcct cctgctcctg cccctcctgc ccctcctgcc cctcctcctg 4020
ctcctgcccc tcctgcccct cctcctgctc ctgcccctcc tcctgctcct gcccctcctg 4080
cccctcctgc ccctcctcct gctcctgccc ctcctcctgc tcctgcccct cctgcccctc 4140
ctgcccctcc tgcccctcct cctgctcctg cccctcctcc tgctcctgcc cctcctgctc 4200
ctgcccctcc cgctcctgct cctgctcctg ttccaccgtg ggtccctttg cagccaatgc 4260
aacttggacg tttttggggt ctccggacac catctctatg tcttggccct gatcctgagc 4320
cgcccggggc tcctggtctt ccgcctcctc gtcctcgtcc tcttccccgt cctcgtccat 4380
ggttatcacc ccctcttctt tgaggtccac tgccgccgga gccttctggt ccagatgtgt 4440
ctcccttctc tcctaggcca tttccaggtc ctgtacctgg cccctcgtca gacatgattc 4500
acactaaaag agatcaatag acatctttat tagacgacgc tcagtgaata cagggagtgc 4560
agactcctgc cccctccaac agccccccca ccctcatccc cttcatggtc gctgtcagac 4620
agatccaggt ctgaaaattc cccatcctcc gaaccatcct cgtcctcatc accaattact 4680
cgcagcccgg aaaactcccg ctgaacatcc tcaagatttg cgtcctgagc ctcaagccag 4740
gcctcaaatt cctcgtcccc ctttttgctg gacggtaggg atggggattc tcgggacccc 4800
tcctcttcct cttcaaggtc accagacaga gatgctactg gggcaacgga agaaaagctg 4860
ggtgcggcct gtgaggatca gcttatcgat gataagctgt caaacatgag aattcttgaa 4920
gacgaaaggg cctcgtgata cgcctatttt tataggttaa tgtcatgata ataatggttt 4980
cttagacgtc aggtggcact tttcggggaa atgtgcgcgg aacccctatt tgtttatttt 5040
tctaaataca ttcaaatatg tatccgctca tgagacaata accctgataa atgcttcaat 5100
aatattgaaa aaggaagagt atgagtattc aacatttccg tgtcgccctt attccctttt 5160
ttgcggcatt ttgccttcct gtttttgctc acccagaaac gctggtgaaa gtaaaagatg 5220
ctgaagatca gttgggtgca cgagtgggtt acatcgaact ggatctcaac agcggtaaga 5280
tccttgagag ttttcgcccc gaagaacgtt ttccaatgat gagcactttt aaagttctgc 5340
tatgtggcgc ggtattatcc cgtgttgacg ccgggcaaga gcaactcggt cgccgcatac 5400
actattctca gaatgacttg gttgagtact caccagtcac agaaaagcat cttacggatg 5460
gcatgacagt aagagaatta tgcagtgctg ccataaccat gagtgataac actgcggcca 5520
acttacttct gacaacgatc ggaggaccga aggagctaac cgcttttttg cacaacatgg 5580
gggatcatgt aactcgcctt gatcgttggg aaccggagct gaatgaagcc ataccaaacg 5640
acgagcgtga caccacgatg cctgcagcaa tggcaacaac gttgcgcaaa ctattaactg 5700
gcgaactact tactctagct tcccggcaac aattaataga ctggatggag gcggataaag 5760

ttgcaggacc acttctgcgc tcggcccttc cggctggctg gtttattgct gataaatctg 5820
gagccggtga gcgtgggtct cgcggtatca ttgcagcact ggggccagat ggtaagccct 5880
cccgtatcgt agttatctac acgacgggga gtcaggcaac tatggatgaa cgaaatagac 5940
agatcgctga gataggtgcc tcactgatta agcattggta actgtcagac caagtttact 6000
catatatact ttagattgat ttaaaacttc atttttaatt taaaaggatc taggtgaaga 6060
tcctttttga taatctcatg accaaaatcc cttaacgtga gttttcgttc cactgagcgt 6120
cagaccccgt agaaaagatc aaaggatctt cttgagatcc tttttttctg cgcgtaatct 6180
gctgcttgca aacaaaaaaa ccaccgctac cagcggtggt ttgtttgccg gatcaagagc 6240
taccaactct ttttccgaag gtaactggct tcagcagagc gcagatacca aatactgtcc 6300
ttctagtgta gccgtagtta ggccaccact tcaagaactc tgtagcaccg cctacatacc 6360
tcgctctgct aatcctgtta ccagtggctg ctgccagtgg cgataagtcg tgtcttaccg 6420
ggttggactc aagacgatag ttaccggata aggcgcagcg gtcgggctga acggggggtt 6480
cgtgcacaca gcccagcttg gagcgaacga cctacaccga actgagatac ctacagcgtg 6540
agctatgaga aagcgccacg cttcccgaag ggagaaaggc ggacaggtat ccggtaagcg 6600
gcagggtcgg aacaggagag cgcacgaggg agcttccagg gggaaacgcc tggtatcttt 6660
atagtcctgt cgggtttcgc cacctctgac ttgagcgtcg atttttgtga tgctcgtcag 6720
gggggcggag cctatggaaa aacgccagca acgcggcctt tttacggttc ctggcctttt 6780
gctgcgccgc gtgcggctgc tggagatggc ggacgcgatg gatatgttct gccaagggtt 6840
ggtttgcgca ttcacagttc tccgcaagaa ttgattggct ccaattcttg gagtggtgaa 6900
tccgttagcg aggtgccgcc ggcttccatt caggtcgagg tggcccggct ccatgcaccg 6960
cgacgcaacg cggggaggca gacaaggtat agggcggcgc ctacaatcca tgccaacccg 7020
ttccatgtgc tcgccgaggc ggcataaatc gccgtgacga tcagcggtcc agtgatcgaa 7080
gttaggctgg taagagccgc gagcgatcct tgaagctgtc cctgatggtc gtcatctacc 7140
tgcctggaca gcatggcctg caacgcgggc atcccgatgc cgccggaagc gagaagaatc 7200
ataatgggga aggccatcca gcctcgcgtc gcgaacgcca gcaagacgta gcccagcgcg 7260
tcggccgcca tgccctgctt catccccgtg gcccgttgct cgcgtttgct ggcggtgtcc 7320
ccggaagaaa tatatttgca tgtctttagt tctatgatga cacaaacccc gcccagcgtc 7380
ttgtcattgg cgaattcgaa cacgcagatg cagtcggggc ggcgcggtcc caggtccact 7440
tcgcatatta aggtgacgcg tgtggcctcg aacaccgagc gaccctgcag cgacccgctt 7500
aacagcgtca acagcgtgcc gcagatcccg ggcaatgaga tatgaaaaag cctgaactca 7560
ccgcgacgtc tgtcgagaag tttctgatcg aaaagttcga cagcgtctcc gacctgatgc 7620
agctctcgga gggcgaagaa tctcgtgctt tcagcttcga tgtaggaggg cgtggatatg 7680
tcctgcgggt aaatagctgc gccgatggtt tctacaaaga tcgttatgtt tatcggcact 7740
ttgcatcggc cgcgctcccg attccggaag tgcttgacat tggggaattc agcgagagcc 7800
tgacctattg catctcccgc cgtgcacagg gtgtcacgtt gcaagacctg cctgaaaccg 7860
aactgcccgc tgttctgcag ccggtcgcgg aggccatgga tgcgatcgct gcggccgatc 7920
ttagccagac gagcgggttc ggcccattcg gaccgcaagg aatcggtcaa tacactacat 7980
ggcgtgattt catatgcgcg attgctgatc cccatgtgta tcactggcaa actgtgatgg 8040
acgacaccgt cagtgcgtcc gtcgcgcagg ctctcgatga gctgatgctt tgggccgagg 8100
actgccccga agtccggcac ctcgtgcacg cggatttcgg ctccaacaat gtcctgacgg 8160
acaatggccg cataacagcg gtcattgact ggagcgaggc gatgttcggg gattcccaat 8220
acgaggtcgc caacatcttc ttctggaggc cgtggttggc ttgtatggag cagcagacgc 8280
gctacttcga gcggaggcat ccggagcttg caggatcgcc gcggctccgg gcgtatatgc 8340
tccgcattgg tcttgaccaa ctctatcaga gcttggttga cggcaatttc gatgatgcag 8400
cttgggcgca gggtcgatgc gacgcaatcg tccgatccgg agccgggact gtcgggcgta 8460
cacaaatcgc ccgcagaagc gcggccgtct ggaccgatgg ctgtgtagaa gtactcgccg 8520
atagtggaaa ccgacgcccc agcactcgtc cgagggcaaa ggaatagggg agatggggga 8580
ggctaactga aacacggaag gagacaatac cggaaggaac ccgcgctatg acggcaataa 8640
aaagacagaa taaaacgcac gggtgttggg tcgtttgttc ataaacgcgg ggttcggtcc 8700
cagggctggc actctgtcga taccccaccg agaccccatt ggggccaata cgcccgcgtt 8760
tcttcctttt ccccacccca ccccccaagt tcgggtgaag gcccagggct cgcagccaac 8820
gtcggggcgg caggccctgc catagccact ggccccgtgg gttagggacg gggtccccca 8880
tggggaatgg tttatggttc gtgggggtta ttattttggg cgttgcgtgg ggtctggtcc 8940
acgactggac tgagcagaca gacccatggt ttttggatgg cctgggcatg gaccgcatgt 9000
actggcgcga cacgaacacc gggcgtctgt ggctgccaaa cacccccgac ccccaaaaac 9060
caccgcgcgg atttctggcg cccagtggtc gaccaattct catgtttgac agcttatcat 9120
cgcagatcct gagcttgtat ggtgcactct cagtacaatc tgctctgctg ccgcatagtt 9180
aagccagtat ctgctccctg cttgtgtgtt ggaggtcgct gagtagtgcg cgagcaaaat 9240
ttaagctaca acaaggcaag gcttgaccga caattgcatg aagaatctgc ttagggttag 9300
gcgttttgcg ctgcttcgcg atgtacgggc cagatatacg cgtatctgag gggactaggg 9360
tgtgtttagg cgcccagcgg ggcttcggtt gtacgcggtt aggagtcccc tcaggatata 9420

gtagtttcgc ttttgcatag ggagggggaa atgtagtctt atgcaataca cttgtagtct 9480
tgcaacatgg taacgatgag ttagcaacat gccttacaag gagagaaaaa gcaccgtgca 9540
tgccgattgg tggaagtaag gtggtacgat cgtgccttat taggaaggca acagacaggt 9600
ctgacatgga ttggacgaac cactgaattc cgcattgcag agataattgt atttaagtgc 9660
ctagctcgat acaataaacg ccatttgacc attcaccaca ttggtgtgca cctccaagct 9720
gggtaccagc tgctagcaag cttccatgga gcccagcagc aagaagctga cgggtcgcct 9780
catgctggcc gtggggggag cagtgcttgg ctccctgcag tttggctaca acactggagt 9840
catcaatgcc ccccagaagg tgatcgagga gttctacaac cagacatggg tccaccgcta 9900
tggggagagc atcctgcccg ccacgctcac cacgctctgg tccctctcag tggccatctt 9960
ttctgttggg ggcatgattg gctccttctc tgtgggcctt ttcgttaacc gctttggccg 10020
gcggaattca atgctgatga tgaacctgct ggccttcgtg tccgccgtgc tcatgggctt 10080
ctcgaaactg ggcaagtcct ttgagatgct gatcctgggc cgcttcatca tcggtgtgta 10140
ctgcggcctg accacaggct tcgtgcccat gtatgtgggt gaagtgtcac ccacagccct 10200
tcgtggggcc ctgggcaccc tgcaccagct gggcatcgtc gtcggcatcc tcatcgccca 10260
ggtgttcggc ctggactcca tcatgggcaa caaggacctg tggcccctgc tgctgagcat 10320
catcttcatc ccggccctgc tgcagtgcat cgtgctgccc ttctgccccg agagtccccg 10380
cttcctgctc atcaaccgca acgaggagaa ccgggccaag agtgtgctaa agaagctgcg 10440
cgggacagct gacgtgaccc atgacctgca ggagatgaag gaagagagtc ggcagatgat 10500
gcgggagaag aaggtcacca tcctggagct gttccgctcc cccgcctacc gccagcccat 10560
cctcatcgct gtggtgctgc agctgtccca gcagctgtct ggcatcaacg ctgtcttcta 10620
ttactccacg agcatcttcg agaaggcggg ggtgcagcag cctgtgtatg ccaccattgg 10680
ctccggtatc gtcaacacgg ccttcactgt tgtgtcgctg tttgtggtgg agcgagcagg 10740
ccggcggacc ctgcatctca taggcctcgc tggcatggcg ggttgtgcca tactcatgac 10800
catcgcgcta gcactgctgg agcagctacc ctggatgtcc tatctgagca tcgtggccat 10860
ctttggcttt gtggccttct ttgaagtggg tcctggcccc atcccatggt tcatcgtggc 10920
tgaactcttc agccagggtc cacgtccagc tgccattgcc gttgcaggct tctccaactg 10980
gacctcaaat ttcattgtgg gcatgtgctt ccagtatgtg gagcaactgt gtggtcccta 11040
cgtcttcatc atcttcactg tgctcctggt tctgttcttc atcttcacct acttcaaagt 11100
tcctgagact aaaggccgga ccttcgatga gatcgcttcc ggcttccggc aggggggagc 11160
cagccaaagt gacaagacac ccgaggagct gttccatccc ctgggggctg attcccaagt 11220
gtgactcgaa attccgcccc tctccctccc ccccccctaa cgttactggc cgaagccgct 11280
tggaataagg ccggtgtgcg tttgtctata tgttattttc caccatattg ccgtcttttg 11340
gcaatgtgag ggcccggaaa cctggccctg tcttcttgac gagcattcct aggggtcttt 11400
cccctctcgc caaaggaatg caaggtctgt tgaatgtcgt gaaggaagca gttcctctgg 11460
aagcttcttg aagacaaaca acgtctgtag cgaccctttg caggcagcgg aaccccccac 11520
ctggcgacag gtgcctctgc ggccaaaagc cacgtgtata agatacacct gcaaaggcgg 11580
cacaacccca gtgccacgtt gtgagttgga tagttgtgga aagagtcaaa tggctctcct 11640
caagcgtatt caacaagggg ctgaaggatg cccagaaggt accccattgt atgggatctg 11700
atctggggcc tcggtgcaca tgctttacat gtgtttagtc gaggttaaaa aacgtctagg 11760
ccccccgaac cacggggacg tggttttcct ttgaaaaaca cgatgataat atggccacaa 11820
ccatggtgag caagggcgag gagctgttca ccggggtggt gcccatcctg gtcgagctgg 11880
acggcgacgt aaacggccac aagttcagcg tgtccggcga gggcgagggc gatgccacct 11940
acggcaagct gaccctgaag ttcatctgca ccaccggcaa gctgcccgtg ccctggccca 12000
ccctcgtgac caccctgacc tacggcgtgc agtgcttcag ccgctacccc gaccacatga 12060
agcagcacga cttcttcaag tccgccatgc ccgaaggcta cgtccaggag cgcaccatct 12120
tcttcaagga cgacggcaac tacaagaccc gcgccgaggt gaagttcgag ggcgacaccc 12180
tggtgaaccg catcgagctg aagggcatcg acttcaagga ggacggcaac atcctggggc 12240
acaagctgga gtacaactac aacagccaca acgtctatat catggccgac aagcagaaga 12300
acggcatcaa ggcgaatttc aagatccgcc acaacatcga ggacggcagc gtgcagctcg 12360
ccgaccacta ccagcagaac acccccatcg gcgacggccc cgtgctgctg cccgacaacc 12420
actacctgag cacccagtcc gccctgagca aagaccccaa cgagaagcgc gatcacatgg 12480
tcctgctgga gttcgtgacc gccgccggga tcactctcgg catggacgag ctgtacaagt 12540
aaagcggcct cgaggccggc aaggccggat ccagacatga taagatacat tgatgagttt 12600
ggacaaacca caactagaat gcagtgaaaa aaatgcttta tttgtgaaat ttgtgatgct 12660
attgctttat ttgtaaccat tataagctgc aataaacaag ttaacaacaa caattgcatt 12720
cattttatgt ttcaggttca gggggaggtg ggaggttttt taaagcaag taaaacctct 12780
acaaatgtgg tatggctgat tatgatccgg ctgcctcgcg cgtttcggtg atgacggtga 12840
aaacctctga cacatgcagc tcccggagac ggtcacagct tgtctgtaag cggatgccgg 12900
gagcagacaa gcccgtcagg gcgcgtcagc gggtgttggc gggtgtcggg gcgcagccat 12960
gaccggtcga ctctaga 12977

<210> 2
<211> 12374
<212> DNA
<213> Künstliche Sequenz

<220>
<223> Beschreibung der künstlichen Sequenz:
      pIJO-25-NeoGFP

<400> 2
gccccgccgc cggacgaact aaacctgact acggcatctc tgccccttct tcgctggtac 60
gaggagcgct tttgttttgt attcggggca gtgcatgtaa tcccttcagt tggttggtac 120
aacttgccaa ctgggccctg ttccacatgt gacacggggg gggaccaaac acaaaggggt 180
tctctgactg tagttgacat ccttataaat ggatgtgcac atttgccaac actgagtggc 240
tttcatcctg gagcagactt tgcagtctgt ggactgcaac acaacattgc ctttatgtgt 300
aactcttggc tgaagctctt acaccaatgc tgggggacat gtacctccca ggggcccagg 360
aagactacgg gaggctacac caacgtcaat cagaggggcc tgtgtagcta ccgataagcg 420
gaccctcaag agggcattag caatagtgtt tataaggccc ccttgttaac cctaaacggg 480
tagcatatgc ttcccgggta gtagtatata ctatccagac taaccctaat tcaatagcat 540
atgttaccca acgggaagca tatgctatcg aattagggtt agtaaaaggg tcctaaggaa 600
cagcgatatc tcccacccca tgagctgtca cggttttatt tacatggggt caggattcca 660
cgagggtagt gaaccatttt agtcacaagg gcagtggctg aagatcaagg agcgggcagt 720
gaactctcct gaatcttcgc ctgcttcttc attctccttc gtttagctaa tagaataact 780
gctgagttgt gaacagtaag gtgtatgtga ggtgctcgaa aacaaggttt caggtgacgc 840
ccccagaata aaatttggac ggggggttca gtggtggcat tgtgctatga caccaatata 900
accctcacaa accccttggg caataaatac tagtgtagga atgaaacatt ctgaatatct 960
ttaacaatag aaatccatgg ggtggggaca agccgtaaag actggatgtc catctcacac 1020
gaatttatgg ctatgggcaa cacataatcc tagtgcaata tgatactggg gttattaaga 1080
tgtgtcccag gcagggacca agacaggtga accatgttgt tacactctat ttgtaacaag 1140
gggaaagaga gtggacgccg acagcagcgg actccactgg ttgtctctaa cacccccgaa 1200
aattaaacgg ggctccacgc caatggggcc cataaacaaa gacaagtggc cactcttttt 1260
tttgaaattg tggagtgggg gcacgcgtca gcccccacac gccgccctgc ggttttggac 1320
tgtaaaataa gggtgtaata acttggctga ttgtaacccc gctaaccact gcggtcaaac 1380
cacttgccca caaaaccact aatggcaccc cggggaatac ctgcataagt aggtgggcgg 1440
gccaagatag gggcgcgatt gctgcgatct ggaggacaaa ttacacacac ttgcgcctga 1500
gcgccaagca cagggttgtt ggtcctcata ttcacgaggt cgctgagagc acggtgggct 1560
aatgttgcca tgggtagcat atactaccca aatatctgga tagcatatgc tatcctaatc 1620
tatatctggg tagcataggc tatcctaatc tatatctggg tagcatatgc tatcctaatc 1680
tatatctggg tagtatatgc tatcctaatt tatatctggg tagcataggc tatcctaatc 1740
tatatctggg tagcatatgc tatcctaatc tatatctggg tagtatatgc tatcctaatc 1800
tgtatccggg tagcatatgc tatcctaata gagattaggg tagtatatgc tatcctaatt 1860
tatatctggg tagcatatac tacccaaata tctggatagc atatgctatc ctaatctata 1920
tctgggtagc atatgctatc ctaatctata tctgggtagc ataggctatc ctaatctata 1980
tctgggtagc atatgctatc ctaatctata tctgggtagt atatgctatc ctaatttata 2040
tctgggtagc ataggctatc ctaatctata tctgggtagc atatgctatc ctaatctata 2100
tctgggtagt atatgctatc ctaatctgta tccgggtagc atatgctatc ctcatgcata 2160
tacagtcagc atatgatacc cagtagtaga gtgggagtgc tatcctttgc atatgccgcc 2220
acctcccaag ggggcgtgaa ttttcgctgc ttgtcctttt cctgcatgct ggttgctccc 2280
attcttaggt gaatttaagg aggccaggct aaagccgtcg catgtctgat tgctcaccag 2340
gtaaatgtcg ctaatgtttt ccaacgcgag aaggtgttga gcgcggagct gagtgacgtg 2400
acaacatggg tatgcccaat tgccccatgt tgggaggacg aaaatggtga caagacagat 2460
ggccagaaat acaccaacag cacgcatgat gtctactggg gatttattct ttagtgcggg 2520
ggaatacacg gcttttaata cgattgaggg cgtctcctaa caagttacat cactcctgcc 2580
cttcctcacc ctcatctcca tcacctcctt catctccgtc atctccgtca tcaccctccg 2640
cggcagcccc ttccaccata ggtggaaacc agggaggcaa atctactcca tcgtcaaagc 2700
tgcacacagt caccctgata ttgcaggtag gagcgggctt tgtcataaca aggtccttaa 2760
tcgcatcctt caaaacctca gcaaatatat gagtttgtaa aaagaccatg aaataacaga 2820
caatggactc ccttagcggg ccaggttgtg ggccgggtcc aggggccatt ccaaagggga 2880
gacgactcaa tggtgtaaga cgacattgtg gaatagcaag ggcagttcct cgccttaggt 2940
tgtaaaggga ggtcttacta cctccatata cgaacacacc ggcgacccaa gttccttcgt 3000

cggtagtcct ttctacgtga ctcctagcca ggagagctct taaaccttct gcaatgttct 3060
caaatttcgg gttggaacct ccttgaccac gatgctttcc aaaccaccct ccttttttgc 3120
gcctgcctcc atcaccctga ccccggggtc cagtgcttgg gccttctcct gggtcatctg 3180
cggggccctg ctctatcgct cccgggggca cgtcaggctc accatctggg ccaccttctt 3240
ggtggtattc aaaataatcg gcttccccta cagggtggaa aaatggcctt ctacctggag 3300
ggggcctgcg cggtggagac ccggatgatg atgactgact actgggactc ctgggcctct 3360
tttctccacg tccacgacct ctccccctgg ctctttcacg acttcccccc ctggctcttt 3420
cacgtcctct accccggcgg cctccactac ctcctcgacc ccggcctcca ctacctcctc 3480
gaccccggcc tccactgcct cctcgacccc ggcctccacc tcctgctcct gcccctcctg 3540
ctcctgcccc tcctcctgct cctgcccctc ctgcccctcc tgctcctgcc cctcctgccc 3600
ctcctgctcc tgcccctcct gcccctcctg ctcctgcccc tcctgcccct cctcctgctc 3660
ctgcccctcc tgcccctcct cctgctcctg cccctcctgc ccctcctgct cctgcccctc 3720
ctgcccctcc tgctcctgcc cctcctgccc ctcctgctcc tgcccctcct gctcctgccc 3780
ctcctgctcc tgcccctcct gctcctgccc ctcctgcccc tcctgcccct cctcctgctc 3840
ctgcccctcc tgctcctgcc cctcctgccc ctcctgcccc tcctgctcct gcccctcctc 3900
ctgctcctgc ccctcctgcc cctcctgccc ctcctcctgc tcctgcccct cctgcccctc 3960
ctcctgctcc tgcccctcct cctgctcctg cccctcctgc ccctcctgcc cctcctcctg 4020
ctcctgcccc tcctgcccct cctcctgctc ctgcccctcc tcctgctcct gcccctcctg 4080
cccctcctgc ccctcctcct gctcctgccc ctcctcctgc tcctgcccct cctgcccctc 4140
ctgcccctcc tgcccctcct cctgctcctg cccctcctcc tgctcctgcc cctcctgctc 4200
ctgcccctcc cgctcctgct cctgctcctg ttccaccgtg ggtccctttg cagccaatgc 4260
aacttggacg tttttggggt ctccggacac catctctatg tcttggccct gatcctgagc 4320
cgcccggggc tcctggtctt ccgcctcctc gtcctcgtcc tcttccccgt cctcgtccat 4380
ggttatcacc ccctcttctt tgaggtccac tgccgccgga gccttctggt ccagatgtgt 4440
ctcccttctc tcctaggcca tttccaggtc ctgtacctgg cccctcgtca gacatgattc 4500
acactaaaag agatcaatag acatctttat tagacgacgc tcagtgaata cagggagtgc 4560
agactcctgc cccctccaac agccccccca ccctcatccc cttcatggtc gctgtcagac 4620
agatccaggt ctgaaaattc cccatcctcc gaaccatcct cgtcctcatc accaattact 4680
cgcagcccgg aaaactcccg ctgaacatcc tcaagatttg cgtcctgagc ctcaagccag 4740
gcctcaaatt cctcgtcccc ctttttgctg gacggtaggg atggggattc tcgggacccc 4800
tcctcttcct cttcaaggtc accagacaga gatgctactg gggcaacgga agaaaagctg 4860
ggtgcggcct gtgaggatca gcttatcgat gataagctgt caaacatgag aattcttgaa 4920
gacgaaaggg cctcgtgata cgcctatttt tataggttaa tgtcatgata ataatggttt 4980
cttagacgtc aggtggcact tttcggggaa atgtgcgcgg aacccctatt tgtttatttt 5040
tctaaataca ttcaaatatg tatccgctca tgagacaata accctgataa atgcttcaat 5100
aatattgaaa aaggaagagt atgagtattc aacatttccg tgtcgccctt attccctttt 5160
ttgcggcatt ttgccttcct gtttttgctc acccagaaac gctggtgaaa gtaaaagatg 5220
ctgaagatca gttgggtgca cgagtgggtt acatcgaact ggatctcaac agcggtaaga 5280
tccttgagag ttttcgcccc gaagaacgtt ttccaatgat gagcactttt aaagttctgc 5340
tatgtggcgc ggtattatcc cgtgttgacg ccgggcaaga gcaactcggt cgccgcatac 5400
actattctca gaatgacttg gttgagtact caccagtcac agaaaagcat cttacggatg 5460
gcatgacagt aagagaatta tgcagtgctg ccataaccat gagtgataac actgcggcca 5520
acttacttct gacaacgatc ggaggaccga aggagctaac cgcttttttg cacaacatgg 5580
gggatcatgt aactcgcctt gatcgttggg aaccggagct gaatgaagcc ataccaaacg 5640
acgagcgtga caccacgatg cctgcagcaa tggcaacaac gttgcgcaaa ctattaactg 5700
gcgaactact tactctagct tcccggcaac aattaataga ctggatggag gcggataaag 5760
ttgcaggacc acttctgcgc tcggcccttc cggctggctg gtttattgct gataaatctg 5820
gagccggtga gcgtgggtct cgcggtatca ttgcagcact ggggccagat ggtaagccct 5880
cccgtatcgt agttatctac acgacgggga gtcaggcaac tatggatgaa cgaaatagac 5940
agatcgctga gataggtgcc tcactgatta agcattggta actgtcagac caagtttact 6000
catatatact ttagattgat ttaaaacttc atttttaatt taaaaggatc taggtgaaga 6060
tcctttttga taatctcatg accaaaatcc cttaacgtga gttttcgttc cactgagcgt 6120
cagaccccgt agaaaagatc aaaggatctt cttgagatcc tttttttctg cgcgtaatct 6180
gctgcttgca aacaaaaaaa ccaccgctac cagcggtggt ttgtttgccg gatcaagagc 6240
taccaactct ttttccgaag gtaactggct tcagcagagc gcagatacca aatactgtcc 6300
ttctagtgta gccgtagtta ggccaccact tcaagaactc tgtagcaccg cctacatacc 6360
tcgctctgct aatcctgtta ccagtggctg ctgccagtgg cgataagtcg tgtcttaccg 6420
ggttggactc aagacgatag ttaccggata aggcgcagcg gtcgggctga acggggggtt 6480
cgtgcacaca gcccagcttg gagcgaacga cctacaccga actgagatac ctacagcgtg 6540
agctatgaga aagcgccacg cttcccgaag ggagaaaggc ggacaggtat ccggtaagcg 6600
gcagggtcgg aacaggagag cgcacgaggg agcttccagg gggaaacgcc tggtatcttt 6660

atagtcctgt cgggtttcgc cacctctgac ttgagcgtcg atttttgtga tgctcgtcag 6720
gggggcggag cctatggaaa aacgccagca acgcggcctt tttacggttc ctggcctttt 6780
gctgcgccgc gtgcggctgc tggagatggc ggacgcgatg gatatgttct gccaagggtt 6840
ggtttgcgca ttcacagttc tccgcaagaa ttgattggct ccaattcttg gagtggtgaa 6900
tccgttagcg aggtgccgcc ggcttccatt caggtcgagg tggcccggct ccatgcaccg 6960
cgacgcaacg cggggaggca gacaaggtat agggcggcgc ctacaatcca tgccaacccg 7020
ttccatgtgc tcgccgaggc ggcataaatc gccgtgacga tcagcggtcc agtgatcgaa 7080
gttaggctgg taagagccgc gagcgatcct tgaagctgtc cctgatggtc gtcatctacc 7140
tgcctggaca gcatggcctg caacgcgggc atcccgatgc cgccggaagc gagaagaatc 7200
ataatgggga aggccatcca gcctcgcgtc gcgaacgcca gcaagacgta gcccagcgcg 7260
tcggccgcca tgccctgctt catccccgtg gcccgttgct cgcgtttgct ggcggtgtcc 7320
ccggaagaaa tatatttgca tgtctttagt tctatgatga cacaaacccc gcccagcgtc 7380
ttgtcattgg cgaattcgaa cacgcagatg cagtcggggc ggcgcggtcc caggtccact 7440
tcgcatatta aggtgacgcg tgtggcctcg aacaccgagc gaccctgcag cgacccgctt 7500
aacagcgtca acagcgtgcc gcagatcccg ggcaatgaga tatgaaaaag cctgaactca 7560
ccgcgacgtc tgtcgagaag tttctgatcg aaaagttcga cagcgtctcc gacctgatgc 7620
agctctcgga gggcgaagaa tctcgtgctt tcagcttcga tgtaggaggg cgtggatatg 7680
tcctgcgggt aaatagctgc gccgatggtt tctacaaaga tcgttatgtt tatcggcact 7740
ttgcatcggc cgcgctcccg attccggaag tgcttgacat tggggaattc agcgagagcc 7800
tgacctattg catctcccgc cgtgcacagg gtgtcacgtt gcaagacctg cctgaaaccg 7860
aactgcccgc tgttctgcag ccggtcgcgg aggccatgga tgcgatcgct gcggccgatc 7920
ttagccagac gagcgggttc ggcccattcg gaccgcaagg aatcggtcaa tacactacat 7980
ggcgtgattt catatgcgcg attgctgatc cccatgtgta tcactggcaa actgtgatgg 8040
acgacaccgt cagtgcgtcc gtcgcgcagg ctctcgatga gctgatgctt tgggccgagg 8100
actgccccga agtccggcac ctcgtgcacg cggatttcgg ctccaacaat gtcctgacgg 8160
acaatggccg cataacagcg gtcattgact ggagcgaggc gatgttcggg gattcccaat 8220
acgaggtcgc caacatcttc ttctggaggc cgtggttggc ttgtatggag cagcagacgc 8280
gctacttcga gcggaggcat ccggagcttg caggatcgcc gcggctccgg gcgtatatgc 8340
tccgcattgg tcttgaccaa ctctatcaga gcttggttga cggcaatttc gatgatgcag 8400
cttgggcgca gggtcgatgc gacgcaatcg tccgatccgg agccgggact gtcgggcgta 8460
cacaaatcgc ccgcagaagc gcggccgtct ggaccgatgg ctgtgtagaa gtactcgccg 8520
atagtggaaa ccgacgcccc agcactcgtc cgagggcaaa ggaatagggg agatggggga 8580
ggctaactga aacacggaag gagacaatac cggaaggaac ccgcgctatg acggcaataa 8640
aaagacagaa taaaacgcac gggtgttggg tcgtttgttc ataaacgcgg ggttcggtcc 8700
cagggctggc actctgtcga taccccaccg agaccccatt ggggccaata cgcccgcgtt 8760
tcttcctttt ccccacccca ccccccaagt tcgggtgaag gcccagggct cgcagccaac 8820
gtcggggcgg caggccctgc catagccact ggccccgtgg gttagggacg gggtccccca 8880
tggggaatgg tttatggttc gtgggggtta ttattttggg cgttgcgtgg ggtctggtcc 8940
acgactggac tgagcagaca gacccatggt ttttggatgg cctgggcatg gaccgcatgt 9000
actggcgcga cacgaacacc gggcgtctgt ggctgccaaa cacccccgac ccccaaaaac 9060
caccgcgcgg atttctggcg cccagtggtc gaccaattct catgtttgac agcttatcat 9120
cgcagatcct gagcttgtat ggtgcactct cagtacaatc tgctctgctg ccgcatagtt 9180
aagccagtat ctgctccctg cttgtgtgtt ggaggtcgct gagtagtgcg cgagcaaaat 9240
ttaagctaca acaaggcaag gcttgaccga caattgcatg aagaatctgc ttagggttag 9300
gcgttttgcg ctgcttcgcg atgtacgggc cagatatacg cgtatctgag gggactaggg 9360
tgtgtttagg cgcccagcgg ggcttcggtt gtacgcggtt aggagtcccc tcaggatata 9420
gtagtttcgc ttttgcatag ggagggggaa atgtagtctt atgcaataca cttgtagtct 9480
tgcaacatgg taacgatgag ttagcaacat gccttacaag gagagaaaaa gcaccgtgca 9540
tgccgattgg tggaagtaag gtggtacgat cgtgccttat taggaaggca acagacaggt 9600
ctgacatgga ttggacgaac cactgaattc cgcattgcag agataattgt atttaagtgc 9660
ctagctcgat acaataaacg ccatttgacc attcaccaca ttggtgtgca cctccaagct 9720
gggtaccagc tgctagcaag cttgctagcg gccgctcgac ctaggctttt gcaaagatcg 9780
atcaagagac aggatgagga tcgtttcgca tgattgaaca agatggattg cacgcaggtt 9840
ctccggccgc ttgggtggag aggctattcg gctatgactg ggcacaacag acaatcggct 9900
gctctgatgc cgccgtgttc cggctgtcag cgcaggggcg cccggttctt tttgtcaaga 9960
ccgacctgtc cggtgccctg aatgaactgc aagacgaggc agcgcggcta tcgtggctgg 10020
ccacgacggg cgttccttgc gcagctgtgc tcgacgttgt cactgaagcg ggaagggact 10080
ggctgctatt gggcgaagtg ccggggcagg atctcctgtc atctcacctt gctcctgccg 10140
agaaagtatc catcatggct gatgcaatgc ggcggctgca tacgcttgat ccggctacct 10200
gcccattcga ccaccaagcg aaacatcgca tcgagcgagc acgtactcgg atggaagccg 10260
gtcttgtcga tcaggatgat ctggacgaag agcatcaggg gctcgcgcca gccgaactgt 10320

tcgccaggct caaggcgagc atgcccgacg gcgaggatct cgtcgtgacc catggcgatg 10380
cctgcttgcc gaatatcatg gtggaaaatg gccgcttttc tggattcatc gactgtggcc 10440
ggctgggtgt ggcggaccgc tatcaggaca tagcgttggc tacccgtgat attgctgaag 10500
agcttggcgg cgaatgggct gaccgcttcc tcgtgcttta cggtatcgcc gctcccgatt 10560
cgcagcgcat cgccttctat cgccttcttg acgagttctt ctgagcggga ctctggggtt 10620
cgtcgaggcc ggcaaggccg gatcaattcc gcccctctcc ctcccccccc cctaacgtta 10680
ctggccgaag ccgcttggaa taaggccggt gtgcgtttgt ctatatgtta ttttccacca 10740
tattgccgtc ttttggcaat gtgagggccc ggaaacctgg ccctgtcttc ttgacgagca 10800
ttcctagggg tctttcccct ctcgccaaag gaatgcaagg tctgttgaat gtcgtgaagg 10860
aagcagttcc tctggaagct tcttgaagac aaacaacgtc tgtagcgacc ctttgcaggc 10920
agcggaaccc cccacctggc gacaggtgcc tctgcggcca aaagccacgt gtataagata 10980
cacctgcaaa ggcggcacaa ccccagtgcc acgttgtgag ttggatagtt gtggaaagag 11040
tcaaatggct ctcctcaagc gtattcaaca aggggctgaa ggatgcccag aaggtacccc 11100
attgtatggg atctgatctg gggcctcggt gcacatgctt tacatgtgtt tagtcgaggt 11160
taaaaaacgt ctaggccccc cgaaccacgg ggacgtggtt ttcctttgaa aaacacgatg 11220
ataatatggc cacaaccatg gtgagcaagg gcgaggagct gttcaccggg gtggtgccca 11280
tcctggtcga gctggacggc gacgtaaacg gccacaagtt cagcgtgtcc ggcgagggcg 11340
agggcgatgc cacctacggc aagctgaccc tgaagttcat ctgcaccacc ggcaagctgc 11400
ccgtgccctg gcccaccctc gtgaccaccc tgacctacgg cgtgcagtgc ttcagccgct 11460
accccgacca catgaagcag cacgacttct tcaagtccgc catgcccgaa ggctacgtcc 11520
aggagcgcac catcttcttc aaggacgacg gcaactacaa gacccgcgcc gaggtgaagt 11580
tcgagggcga caccctggtg aaccgcatcg agctgaaggg catcgacttc aaggaggacg 11640
gcaacatcct ggggcacaag ctggagtaca actacaacag ccacaacgtc tatatcatgg 11700
ccgacaagca gaagaacggc atcaaggcga atttcaagat ccgccacaac atcgaggacg 11760
gcagcgtgca gctcgccgac cactaccagc agaacacccc catcggcgac ggccccgtgc 11820
tgctgcccga caaccactac ctgagcaccc agtccgccct gagcaaagac cccaacgaga 11880
agcgcgatca catggtcctg ctggagttcg tgaccgccgc cgggatcact ctcggcatgg 11940
acgagctgta caagtaaagc ggccgatcca gacatgataa gatacattga tgagtttgga 12000
caaaccacaa ctagaatgca gtgaaaaaaa tgctttattt gtgaaatttg tgatgctatt 12060
gctttatttg taaccattat aagctgcaat aaacaagtta acaacaacaa ttgcattcat 12120
tttatgtttc aggttcaggg ggaggtgggg aggtttttta aagcaagtaa aacctctaca 12180
aatgtggtat ggctgattat gatccggctg cctcgcgcgt ttcggtgatg acggtgaaaa 12240
cctctgacac atgcagctcc cggagacggt cacagcttgt ctgtaagcgg atgccgggag 12300
cagacaagcc cgtcagggcg cgtcagcggg tgttggcggg tgtcggggcg cagccatgac 12360
cggtcgactc taga 12374

<210> 3
<211> 11687
<212> DNA
<213> Künstliche Sequenz

<220>
<223> Beschreibung der künstlichen Sequenz: pIJO-76
(GLUT-PV-SEAP)

<400> 3
tagttattaa tagtaatcaa ttacggggtc attagttcat agcccatata tggagttccg 60
cgttacataa cttacggtaa atggcccgcc tggctgaccg cccaacgacc cccgcccatt 120
gacgtcaata atgacgtatg ttcccatagt aacgccaata gggactttcc attgacgtca 180
atgggtggag tatttacggt aaactgccca cttggcagta catcaagtgt atcatatgcc 240
aagtacgccc cctattgacg tcaatgacgg taaatggccc gcctggcatt atgcccagta 300
catgacctta tgggactttc ctacttggca gtacatctac gtattagtca tcgctattac 360
catggtgatg cggttttggc agtacatcaa tgggcgtgga tagcggtttg actcacgggg 420
atttccaagt ctccacccca ttgacgtcaa tgggagtttg ttttggcacc aaaatcaacg 480
ggactttcca aaatgtcgta acaactccgc cccattgacg caaatgggcg gtaggcgtgt 540
acggtgggag gtctatataa gcagagctgg tttagtgaac cgtcagatcc gctagcgcta 600
ccggactcag atccccataa gcttccatgg agcccagcag caagaagctg acgggtcgcc 660
tcatgctggc cgtgggagga gcagtgcttg gctccctgca gtttggctac aacactggag 720
tcatcaatgc cccccagaag gtgatcgagg agttctacaa ccagacatgg gtccaccgct 780
atggggagag catcctgccc accacgctca ccacgctctg gtccctctca gtggccatct 840

tttctgttgg gggcatgatt ggctccttct ctgtgggcct tttcgttaac cgctttggcc 900
ggcggaattc aatgctgatg atgaacctgc tggccttcgt gtccgccgtg ctcatgggct 960
tctcgaaact gggcaagtcc tttgagatgc tgatcctggg ccgcttcatc atcggtgtgt 1020
actgcggcct gaccacaggc ttcgtgccca tgtatgtggg tgaagtgtca cccacagccc 1080
ttcgtggggc cctgggcacc ctgcaccagc tgggcatcgt cgtcggcatc ctcatcgccc 1140
aggtgttcgg cctggactcc atcatgggca acaaggacct gtggcccctg ctgctgagca 1200
tcatcttcat cccggccctg ctgcagtgca tcgtgctgcc cttctgcccc gagagtcccc 1260
gcttcctgct catcaaccgc aacgaggaga accgggccaa gagtgtgcta aagaagctgc 1320
gcgggacagc tgacgtgacc catgacctgc aggagatgaa ggaagagagt cggcagatga 1380
tgcgggagaa gaaggtcacc atcctggagc tgttccgctc ccccgcctac cgccagccca 1440
tcctcatcgc tgtggtgctg cagctgtccc agcagctgtc tggcatcaac gctgtcttct 1500
attactccac gagcatcttc gagaaggcgg gggtgcagca gcctgtgtat gccaccattg 1560
gctccggtat cgtcaacacg gccttcactg ttgtgtcgct gtttgtggtg gagcgagcag 1620
gccggcggac cctgcatctc ataggcctcg ctggcatggc gggttgtgcc atactcatga 1680
ccatcgcgct agcactgctg gagcagctac cctggatgtc ctatctgagc atcgtggcca 1740
tctttggctt tgtggccttc tttgaagtgg gtcctggccc catcccatgg ttcatcgtgg 1800
ctgaactctt cagccagggt ccacgtccag ctgccattgc cgttgcaggc ttctccaact 1860
ggacctcaaa tttcattgtg ggcatgtgct tccagtatgt ggagcaactg tgtggtccct 1920
acgtcttcat catcttcact gtgctcctgg ttctgttctt catcttcacc tacttcaaag 1980
ttcctgagac taaaggccgg accttcgatg agatcgcttc cggcttccgg caggggggag 2040
ccagccaaag tgacaagaca cccgaggagc tgttccatcc cctgggggct gattcccaag 2100
tgtgactcga cggtaccgcg ggcccgggat ccccgggtac cggaattccg cccctctccc 2160
tccccccccc ctaacgttac tggccgaagc cgcttggaat aaggccggtg tgcgtttgtc 2220
tatatgttat tttccaccat attgccgtct tttggcaatg tgagggcccg gaaacctggc 2280
cctgtcttct tgacgagcat tcctaggggt ctttcccctc tcgccaaagg aatgcaaggt 2340
ctgttgaatg tcgtgaagga agcagttcct ctggaagctt cttgaagaca aacaacgtct 2400
gtagcgaccc tttgcaggca gcggaacccc ccacctggcg acaggtgcct ctgcggccaa 2460
aagccacgtg tataagatac acctgcaaag gcggcacaac cccagtgcca cgttgtgagt 2520
tggatagttg tggaaagagt caaatggctc tcctcaagcg tattcaacaa ggggctgaag 2580
gatgcccaga aggtacccca ttgtatggga tctgatctgg ggcctcggtg cacatgcttt 2640
acatgtgttt agtcgaggtt aaaaaacgtc taggcccccc gaaccacggg gacgtggttt 2700
tcctttgaaa aacacgatga taatatggcc acaaccatgg tgagcaaggg cgaggagctg 2760
ttcaccgggg tggtgcccat cctggtcgag ctggacggcg acgtaaacgg ccacaagttc 2820
agcgtgtccg gcgagggcga gggcgatgcc acctacggca agctgaccct gaagttcatc 2880
tgcaccaccg gcaagctgcc cgtgccctgg cccaccctcg tgaccaccct gacctacggc 2940
gtgcagtgct tcagccgcta ccccgaccac atgaagcagc acgacttctt caagtccgcc 3000
atgcccgaag gctacgtcca ggagcgcacc atcttcttca aggacgacgg caactacaag 3060
acccgcgccg aggtgaagtt cgagggcgac accctggtga accgcatcga gctgaagggc 3120
atcgacttca aggaggacgg caacatcctg gggcacaagc tggagtacaa ctacaacagc 3180
cacaacgtct atatcatggc cgacaagcag aagaacggca tcaaggcgaa tttcaagatc 3240
cgccacaaca tcgaggacgg cagcgtgcag ctcgccgacc actaccagca gaacaccccc 3300
atcggcgacg gccccgtgct gctgcccgac aaccactacc tgagcaccca gtccgccctg 3360
agcaaagacc ccaacgagaa gcgcgatcac atggtcctgc tggagttcgt gaccgccgcc 3420
gggatcactc tcggcatgga cgagctgtac aagtaaagcg gccgcgactc tagatcataa 3480
tcagccatac cacatttgta gaggttttac ttgctttaaa aaacctccca cacctccccc 3540
tgaacctgaa acataaaatg aatgcaattg ttgttgttaa cttgtttatt gcagcttata 3600
atggttacaa ataaagcaat agcatcacaa atttcacaaa taaagcattt ttttcactgc 3660
attctagttg tggtttgtcc aaactcatca atgtatctta aggcgtaaat tgtaagcgtt 3720
aatattttgt taaaattcgc gttaaatttt tgttaaatca gctcattttt taaccaatag 3780
gccgaaatcg gcaaaatccc ttataaatca aaagaataga ccgagatagg gttgagtgtt 3840
gttccagttt ggaacaagag tccactatta aagaacgtgg actccaacgt caaagggcga 3900
aaaaccgtct atcagggcga tggcccactc gactctagag ccccgccgcc ggacgaacta 3960
aacctgacta cggcatctct gccccttctt cgctggtacg aggagcgctt ttgttttgta 4020
ttcggggcag tgcatgtaat cccttcagtt ggttggtaca acttgccaac tgggccctgt 4080
tccacatgtg acacgggggg ggaccaaaca caaaggggtt ctctgactgt agttgacatc 4140
cttataaatg gatgtgcaca tttgccaaca ctgagtggct ttcatcctgg agcagacttt 4200
gcagtctgtg gactgcaaca caacattgcc tttatgtgta actcttggct gaagctctta 4260
caccaatgct gggggacatg tacctcccag gggcccagga agactacggg aggctacacc 4320
aacgtcaatc agaggggcct gtgtagctac cgataagcgg accctcaaga gggcattagc 4380
aatagtgttt ataaggcccc cttgttaacc ctaaacgggt agcatatgct tcccgggtag 4440
tagtatatac tatccagact aaccctaatt caatagcata tgttacccaa cgggaagcat 4500

atgctatcga attagggtta gtaaaagggt cctaaggaac agcgatatct cccaccccat 4560
gagctgtcac ggttttattt acatggggtc aggattccac gagggtagtg aaccatttta 4620
gtcacaaggg cagtggctga agatcaagga gcgggcagtg aactctcctg aatcttcgcc 4680
tgcttcttca ttctccttcg tttagctaat agaataactg ctgagttgtg aacagtaagg 4740
tgtatgtgag gtgctcgaaa acaaggtttc aggtgacgcc cccagaataa aatttggacg 4800
gggggttcag tggtggcatt gtgctatgac accaatataa ccctcacaaa ccccttgggc 4860
aataaatact agtgtaggaa tgaaacattc tgaatatctt taacaataga aatccatggg 4920
gtggggacaa gccgtaaaga ctggatgtcc atctcacacg aatttatggc tatgggcaac 4980
acataatcct agtgcaatat gatactgggg ttattaagat gtgtcccagg cagggaccaa 5040
gacaggtgaa ccatgttgtt acactctatt tgtaacaagg ggaaagagag tggacgccga 5100
cagcagcgga ctccactggt tgtctctaac acccccgaaa attaaacggg gctccacgcc 5160
aatggggccc ataaacaaag acaagtggcc actctttttt ttgaaattgt ggagtggggg 5220
cacgcgtcag cccccacacg ccgccctgcg gttttggact gtaaaataag ggtgtaataa 5280
cttggctgat tgtaaccccg ctaaccactg cggtcaaacc acttgcccac aaaaccacta 5340
atggcacccc ggggaatacc tgcataagta ggtgggcggg ccaagatagg ggcgcgattg 5400
ctgcgatctg gaggacaaat tacacacact tgcgcctgag cgccaagcac agggttgttg 5460
gtcctcatat tcacgaggtc gctgagagca cggtgggcta atgttgccat gggtagcata 5520
tactacccaa atatctggat agcatatgct atcctaatct atatctgggt agcataggct 5580
atcctaatct atatctgggt agcatatgct atcctaatct atatctgggt agtatatgct 5640
atcctaattt atatctgggt agcataggct atcctaatct atatctgggt agcatatgct 5700
atcctaatct atatctgggt agtatatgct atcctaatct gtatccgggt agcatatgct 5760
atcctaatag agattagggt agtatatgct atcctaattt atatctgggt agcatatact 5820
acccaaatat ctggatagca tatgctatcc taatctatat ctgggtagca tatgctatcc 5880
taatctatat ctgggtagca taggctatcc taatctatat ctgggtagca tatgctatcc 5940
taatctatat ctgggtagta tatgctatcc taatttatat ctgggtagca taggctatcc 6000
taatctatat ctgggtagca tatgctatcc taatctatat ctgggtagta tatgctatcc 6060
taatctgtat ccgggtagca tatgctatcc tcatgcatat acagtcagca tatgataccc 6120
agtagtagag tgggagtgct atcctttgca tatgccgcca cctcccaagg gggcgtgaat 6180
tttcgctgct tgtccttttc ctgcatgctg gttgctccca ttcttaggtg aatttaagga 6240
ggccaggcta aagccgtcgc atgtctgatt gctcaccagg taaatgtcgc taatgttttc 6300
caacgcgaga aggtgttgag cgcggagctg agtgacgtga caacatgggt atgcccaatt 6360
gccccatgtt gggaggacga aaatggtgac aagacagatg gccagaaata caccaacagc 6420
acgcatgatg tctactgggg atttattctt tagtgcgggg gaatacacgg cttttaatac 6480
gattgagggc gtctcctaac aagttacatc actcctgccc ttcctcaccc tcatctccat 6540
cacctccttc atctccgtca tctccgtcat caccctccgc ggcagcccct tccaccatag 6600
gtggaaacca gggaggcaaa tctactccat cgtcaaagct gcacacagtc accctgatat 6660
tgcaggtagg agcgggcttt gtcataacaa ggtccttaat cgcatccttc aaaacctcag 6720
caaatatatg agtttgtaaa aagaccatga aataacagac aatggactcc cttagcgggc 6780
caggttgtgg gccgggtcca ggggccattc caaaggggag acgactcaat ggtgtaagac 6840
gacattgtgg aatagcaagg gcagttcctc gccttaggtt gtaaagggag gtcttactac 6900
ctccatatac gaacacaccg gcgacccaag ttccttcgtc ggtagtcctt tctacgtgac 6960
tcctagccag gagagctctt aaaccttctg caatgttctc aaatttcggg ttggaacctc 7020
cttgaccacg atgctttcca aaccaccctc cttttttgcg cctgcctcca tcaccctgac 7080
cccggggtcc agtgcttggg ccttctcctg ggtcatctgc ggggccctgc tctatcgctc 7140
ccgggggcac gtcaggctca ccatctgggc caccttcttg gtggtattca aaataatcgg 7200
cttcccctac agggtggaaa aatggccttc tacctggagg gggcctgcgc ggtggagacc 7260
cggatgatga tgactgacta ctgggactcc tgggcctctt ttctccacgt ccacgacctc 7320
tccccctggc tctttcacga cttccccccc tggctctttc acgtcctcta ccccggcggc 7380
ctccactacc tcctcgaccc cggcctccac tacctcctcg accccggcct ccactgcctc 7440
ctcgaccccg gcctccacct cctgctcctg cccctcctgc tcctgcccct cctcctgctc 7500
ctgcccctcc tgcccctcct gctcctgccc ctcctgcccc tcctgctcct gcccctcctg 7560
cccctcctgc tcctgcccct cctgcccctc ctcctgctcc tgcccctcct gcccctcctc 7620
ctgctcctgc ccctcctgcc cctcctgctc ctgcccctcc tgcccctcct gctcctgccc 7680
ctcctgcccc tcctgctcct gcccctcctg ctcctgcccc tcctgctcct gcccctcctg 7740
ctcctgcccc tcctgcccct cctgcccctc ctcctgctcc tgcccctcct gctcctgccc 7800
ctcctgcccc tcctgcccct cctgctcctg cccctcctcc tgctcctgcc cctcctgccc 7860
ctcctgcccc tcctcctgct cctgcccctc ctgcccctcc tcctgctcct gcccctcctc 7920
ctgctcctgc ccctcctgcc cctcctgccc ctcctcctgc tcctgcccct cctgcccctc 7980
ctcctgctcc tgcccctcct cctgctcctg cccctcctgc ccctcctgcc cctcctcctg 8040
ctcctgcccc tcctcctgct cctgcccctc ctgcccctcc tgcccctcct gcccctcctc 8100
ctgctcctgc ccctcctcct gctcctgccc ctcctgctcc tgcccctccc gctcctgctc 8160

ctgctcctgt tccaccgtgg gtccctttgc agccaatgca acttggacgt ttttggggtc 8220
tccggacacc atctctatgt cttggccctg atcctgagcc gcccggggct cctggtcttc 8280
cgcctcctcg tcctcgtcct cttccccgtc ctcgtccatg gttatcaccc cctcttcttt 8340
gaggtccact gccgccggag ccttctggtc cagatgtgtc tcccttctct cctaggccat 8400
ttccaggtcc tgtacctggc ccctcgtcag acatgattca cactaaaaga gatcaataga 8460
catctttatt agacgacgct cagtgaatac agggagtgca gactcctgcc ccctccaaca 8520
gcccccccac cctcatcccc ttcatggtcg ctgtcagaca gatccaggtc tgaaaattcc 8580
ccatcctccg aaccatcctc gtcctcatca ccaattactc gcagcccgga aaactcccgc 8640
tgaacatcct caagatttgc gtcctgagcc tcaagccagg cctcaaattc ctcgtccccc 8700
tttttgctgg acggtaggga tggggattct cgggacccct cctcttcctc ttcaaggtca 8760
ccagacagag atgctactgg ggcaacggaa gaaaagctgg gtgcggcctg tgaggatcag 8820
cttatcggtg aaccatcacc ctaatcaagt tttttggggt cgaggtgccg taaagcacta 8880
aatcggaacc ctaaagggag cccccgattt agagcttgac ggggaaagcc ggcgaacgtg 8940
gcgagaaagg aagggaagaa agcgaaagga gcgggcgcta gggcgctggc aagtgtagcg 9000
gtcacgctgc gcgtaaccac cacacccgcc gcgcttaatg cgccgctaca gggcgcgtca 9060
ggtggcactt ttcggggaaa tgtgcgcgga acccctattt gtttattttt ctaaatacat 9120
tcaaatatgt atccgctcat gagacaataa ccctgataaa tgcttcaata atattgaaaa 9180
aggaagagtc ctgaggcgga aagaaccagc tgtggaatgt gtgtcagtta gggtgtggaa 9240
agtccccagg ctccccagca ggcagaagta tgcaaagcat gcatctcaat tagtcagcaa 9300
ccaggtgtgg aaagtcccca ggctccccag caggcagaag tatgcaaagc atgcatctca 9360
attagtcagc aaccatagtc ccgcccctaa ctccgcccat cccgccccta actccgccca 9420
gttccgccca ttctccgccc catggctgac taattttttt tatttatgca gaggccgagg 9480
ccgcctcggc ctctgagcta ttccagaagt agtgaggagg cttttttgga ggcctaggct 9540
tttgcaaaga tcgatcaaga gacaggatga ggatcgtttc gcatgattga acaagatgga 9600
ttgcacgcag gttctccggc cgcttgggtg gagaggctat tcggctatga ctgggcacaa 9660
cagacaatcg gctgctctga tgccgccgtg ttccggctgt cagcgcaggg gcgcccggtt 9720
ctttttgtca agaccgacct gtccggtgcc ctgaatgaac tgcaagacga ggcagcgcgg 9780
ctatcgtggc tggccacgac gggcgttcct tgcgcagctg tgctcgacgt tgtcactgaa 9840
gcgggaaggg actggctgct attgggcgaa gtgccggggc aggatctcct gtcatctcac 9900
cttgctcctg ccgagaaagt atccatcatg gctgatgcaa tgcggcggct gcatacgctt 9960
gatccggcta cctgcccatt cgaccaccaa gcgaaacatc gcatcgagcg agcacgtact 10020
cggatggaag ccggtcttgt cgatcaggat gatctggacg aagagcatca ggggctcgcg 10080
ccagccgaac tgttcgccag gctcaaggcg agcatgcccg acggcgagga tctcgtcgtg 10140
acccatggcg atgcctgctt gccgaatatc atggtggaaa atggccgctt ttctggattc 10200
atcgactgtg gccggctggg tgtggcggac cgctatcagg acatagcgtt ggctacccgt 10260
gatattgctg aagagcttgg cggcgaatgg gctgaccgct tcctcgtgct ttacggtatc 10320
gccgctcccg attcgcagcg catcgccttc tatcgccttc ttgacgagtt cttctgagcg 10380
ggactctggg gttcgaaatg accgaccaag cgacgcccaa cctgccatca cgagatttcg 10440
attccaccgc cgccttctat gaaaggttgg gcttcggaat cgttttccgg gacgccggct 10500
ggatgatcct ccagcgcggg gatctcatgc tggagttctt cgcccaccct agggggaggc 10560
taactgaaac acggaaggag acaataccgg aaggaacccg cgctatgacg gcaataaaaa 10620
gacagaataa aacgcacggt gttgggtcgt ttgttcataa acgcggggtt cggtcccagg 10680
gctggcactc tgtcgatacc ccaccgagac cccattgggg ccaatacgcc cgcgtttctt 10740
ccttttcccc accccacccc ccaagttcgg gtgaaggccc agggctcgca gccaacgtcg 10800
gggcggcagg ccctgccata gcctcaggtt actcatatat actttagatt gatttaaaac 10860
ttcattttta atttaaaagg atctaggtga agatcctttt tgataatctc atgaccaaaa 10920
tcccttaacg tgagttttcg ttccactgag cgtcagaccc cgtagaaaag atcaaaggat 10980
cttcttgaga tccttttttt ctgcgcgtaa tctgctgctt gcaaacaaaa aaaccaccgc 11040
taccagcggt ggtttgtttg ccggatcaag agctaccaac tctttttccg aaggtaactg 11100
gcttcagcag agcgcagata ccaaatactg tccttctagt gtagccgtag ttaggccacc 11160
acttcaagaa ctctgtagca ccgcctacat acctcgctct gctaatcctg ttaccagtgg 11220
ctgctgccag tggcgataag tcgtgtctta ccgggttgga ctcaagacga tagttaccgg 11280
ataaggcgca gcggtcgggc tgaacggggg gttcgtgcac acagcccagc ttggagcgaa 11340
cgacctacac cgaactgaga tacctacagc gtgagctatg agaaagcgcc acgcttcccg 11400
aagggagaaa ggcggacagg tatccggtaa gcggcagggt cggaacagga gagcgcacga 11460
gggagcttcc agggggaaac gcctggtatc tttatagtcc tgtcgggttt cgccacctct 11520
gacttgagcg tcgatttttg tgatgctcgt caggggggcg gagcctatgg aaaaacgcca 11580
gcaacgcggc ctttttacgg ttcctggcct tttgctggcc ttttgctcac atgttctttc 11640
ctgcgttatc ccctgattct gtggataacc gtattaccgc catgcat 11687

<210> 4
<211> 11200
<212> DNA
<213> Künstliche Sequenz

<220>
<223> Beschreibung der künstlichen Sequenz: pIJO-73
      (PV-SEAP)

<400> 4
tagttattaa tagtaatcaa ttacggggtc attagttcat agcccatata tggagttccg 60
cgttacataa cttacggtaa atggcccgcc tggctgaccg cccaacgacc cccgcccatt 120
gacgtcaata atgacgtatg ttcccatagt aacgccaata gggactttcc attgacgtca 180
atgggtggag tatttacggt aaactgccca cttggcagta catcaagtgt atcatatgcc 240
aagtacgccc cctattgacg tcaatgacgg taaatggccc gcctggcatt atgcccagta 300
catgacctta tgggactttc ctacttggca gtacatctac gtattagtca tcgctattac 360
catggtgatg cggttttggc agtacatcaa tgggcgtgga tagcggtttg actcacgggg 420
atttccaagt ctccacccca ttgacgtcaa tgggagtttg ttttggcacc aaaatcaacg 480
ggactttcca aaatgtcgta acaactccgc cccattgacg caaatgggcg gtaggcgtgt 540
acggtgggag gtctatataa gcagagctgg tttagtgaac cgtcagatcc gctagcgcta 600
ccggactcag atctcgaggt cgacggtatc gataagctta aaacagctct ggggttgtac 660
ccaccccaga ggcccacgtg gcggctagta ctccggtatt gcggtaccct tgtacgcctg 720
ttttatactc ccttcccgta acttagacgc acaaaaccaa gttcaataga agggggtaca 780
aaccagtacc accacgaaca agcacttctg tttccccggt gatgtcgtat agactgcttg 840
cgtggttgaa agcgacggat ccgttatccg cttatgtact tcgagaagcc cagtaccacc 900
tcggaatctt cgatgcgttg cgctcagcac tcaaccccag agtgtagctt aggctgatga 960
gtctggacat ccctcaccgg tgacggtggt ccaggctgcg ttggcggcct acctatggct 1020
aacgccatgg gacgctagtt gtgaacaagg tgtgaagagc ctattgagct acataagaat 1080
cctccggccc ctgaatgcgg ctaatcccaa cctcggagca ggtggtcaca aaccagtgat 1140
tggcctgtcg taacgcgcaa gtccgtggcg gaaccgacta ctttgggtgt ccgtgtttcc 1200
ttttatttta ttgtggctgc ttatggtgac aatcacagat tgttatcata aagcgaattg 1260
gattgcggcc gtcgacgctt gttctttttg cagaagctca gaataaacgc tcaactagct 1320
tgatatcgaa ttcctgcagc ccagcttcct gcatgctgct gctgctgctg ctgctgggcc 1380
tgaggctaca gctctccctg ggcatcatcc cagttgagga ggagaacccg gacttctgga 1440
accgcgaggc agccgaggcc ctgggtgccg ccaagaagct gcagcctgca cagacagccg 1500
ccaagaacct catcatcttc ctgggcgatg ggatgggggt gtctacggtg acagctgcca 1560
ggatcctaaa agggcagaag aaggacaaac tggggcctga gatacccctg gccatggacc 1620
gcttcccata tgtggctctg tccaagacat acaatgtaga caaacatgtg ccagacagtg 1680
gagccacagc cacggcctac ctgtgcgggg tcaagggcaa cttccagacc attggcttga 1740
gtgcagccgc ccgctttaac cagtgcaaca cgacacgcgg caacgaggtc atctccgtga 1800
tgaatcgggc caagaaagca gggaagtcag tgggagtggt aaccaccaca cgagtgcagc 1860
acgcctcgcc agccggcacc tacgcccaca cggtgaaccg caactggtac tcggacgccg 1920
acgtgcctgc ctccgcccgc caggaggggt gccaggacat cgctacgcag ctcatctcca 1980
acatggacat tgacgtgatc ctaggtggag gccgaaagta catgtttcgc atgggaaccc 2040
cagaccctga gtacccagat gactacagcc aaggtgggac caggctggac gggaagaatc 2100
tggtgcagga atggctggcg aagcgccagg gtgcccggta tgtgtggaac cgcactgagc 2160
tcatgcaggc ttccctggac ccgtctgtga cccatctcat gggtctcttt gagcctggag 2220
acatgaaata cgagatccac cgagactcca cactggaccc ctccctgatg gagatgacag 2280
aggctgccct gcgcctgctg agcaggaacc cccgcggctt cttcctcttc gtggagggtg 2340
gtcgcatcga ccatggtcat catgaaagca gggcttaccg ggcactgact gagacgatca 2400
tgttcgacga cgccattgag agggcgggcc agctcaccag cgaggaggac acgctgagcc 2460
tcgtcactgc cgaccactcc cacgtcttct ccttcggagg ctaccccctg cgagggagct 2520
ccatcttcgg gctggcccct ggcaaggccc gggacaggaa ggcctacacg gtcctcctat 2580
acggaaacgg tccaggctat gtgctcaagg acggcgcccg gccggatgtt accgagagcg 2640
agagcgggag ccccgagtat cggcagcagt cagcagtgcc cctggacgaa gagacccacg 2700
caggcgagga cgtggcggtg ttcgcgcgcg gcccgcaggc gcacctggtt cacggcgtgc 2760
aggagcagac cttcatagcg cacgtcatgg ccttcgccgc ctgcctggag ccctacaccg 2820
cctgcgacct ggcgcccccc gccggcacca ccgacgccgc gcacccgggt taacccgtgg 2880
tccccgcgtt gcttcctctg ctggccggga catcaggtgg cccccgctga attggaatcg 2940
atcagaattc tgcagatatc catcacactg gcggccgcga ctctagatca taatcagcca 3000
taccacattt gtagaggttt tacttgcttt aaaaaacctc ccacacctcc ccctgaacct 3060

gaaacataaa atgaatgcaa ttgttgttgt taacttgttt attgcagctt ataatggtta 3120
caaataaagc aatagcatca caaatttcac aaataaagca tttttttcac tgcattctag 3180
ttgtggtttg tccaaactca tcaatgtatc ttaaggcgta aattgtaagc gttaatattt 3240
tgttaaaatt cgcgttaaat ttttgttaaa tcagctcatt ttttaaccaa taggccgaaa 3300
tcggcaaaat cccttataaa tcaaaagaat agaccgagat agggttgagt gttgttccag 3360
tttggaacaa gagtccacta ttaaagaacg tggactccaa cgtcaaaggg cgaaaaaccg 3420
tctatcaggg cgatggccca ctcgactcta gagccccgcc gccggacgaa ctaaacctga 3480
ctacggcatc tctgcccctt cttcgctggt acgaggagcg cttttgtttt gtattcgggg 3540
cagtgcatgt aatcccttca gttggttggt acaacttgcc aactgggccc tgttccacat 3600
gtgacacggg gggggaccaa acacaaaggg gttctctgac tgtagttgac atccttataa 3660
atggatgtgc acatttgcca acactgagtg gctttcatcc tggagcagac tttgcagtct 3720
gtggactgca acacaacatt gcctttatgt gtaactcttg gctgaagctc ttacaccaat 3780
gctgggggac atgtacctcc caggggccca ggaagactac gggaggctac accaacgtca 3840
atcagagggg cctgtgtagc taccgataag cggaccctca agagggcatt agcaatagtg 3900
tttataaggc ccccttgtta accctaaacg ggtagcatat gcttcccggg tagtagtata 3960
tactatccag actaacccta attcaatagc atatgttacc caacgggaag catatgctat 4020
cgaattaggg ttagtaaaag ggtcctaagg aacagcgata tctcccaccc catgagctgt 4080
cacggtttta tttacatggg gtcaggattc cacgagggta gtgaaccatt ttagtcacaa 4140
gggcagtggc tgaagatcaa ggagcgggca gtgaactctc ctgaatcttc gcctgcttct 4200
tcattctcct tcgtttagct aatagaataa ctgctgagtt gtgaacagta aggtgtatgt 4260
gaggtgctcg aaaacaaggt ttcaggtgac gcccccagaa taaaatttgg acggggggtt 4320
cagtggtggc attgtgctat gacaccaata taaccctcac aaaccccttg ggcaataaat 4380
actagtgtag gaatgaaaca ttctgaatat ctttaacaat agaaatccat ggggtgggga 4440
caagccgtaa agactggatg tccatctcac acgaatttat ggctatgggc aacacataat 4500
cctagtgcaa tatgatactg gggttattaa gatgtgtccc aggcagggac caagacaggt 4560
gaaccatgtt gttacactct atttgtaaca aggggaaaga gagtggacgc cgacagcagc 4620
ggactccact ggttgtctct aacacccccg aaaattaaac ggggctccac gccaatgggg 4680
cccataaaca aagacaagtg gccactcttt tttttgaaat tgtggagtgg gggcacgcgt 4740
cagcccccac acgccgccct gcggttttgg actgtaaaat aagggtgtaa taacttggct 4800
gattgtaacc ccgctaacca ctgcggtcaa accacttgcc cacaaaacca ctaatggcac 4860
cccggggaat acctgcataa gtaggtgggc gggccaagat aggggcgcga ttgctgcgat 4920
ctggaggaca aattacacac acttgcgcct gagcgccaag cacagggttg ttggtcctca 4980
tattcacgag gtcgctgaga gcacggtggg ctaatgttgc catgggtagc atatactacc 5040
caaatatctg gatagcatat gctatcctaa tctatatctg ggtagcatag gctatcctaa 5100
tctatatctg ggtagcatat gctatcctaa tctatatctg ggtagtatat gctatcctaa 5160
tttatatctg ggtagcatag gctatcctaa tctatatctg ggtagcatat gctatcctaa 5220
tctatatctg ggtagtatat gctatcctaa tctgtatccg ggtagcatat gctatcctaa 5280
tagagattag ggtagtatat gctatcctaa tttatatctg ggtagcatat actacccaaa 5340
tatctggata gcatatgcta tcctaatcta tatctgggta gcatatgcta tcctaatcta 5400
tatctgggta gcataggcta tcctaatcta tatctgggta gcatatgcta tcctaatcta 5460
tatctgggta gtatatgcta tcctaattta tatctgggta gcataggcta tcctaatcta 5520
tatctgggta gcatatgcta tcctaatcta tatctgggta gtatatgcta tcctaatctg 5580
tatccgggta gcatatgcta tcctcatgca tatacagtca gcatatgata cccagtagta 5640
gagtgggagt gctatccttt gcatatgccg ccacctccca agggggcgtg aattttcgct 5700
gcttgtcctt ttcctgcatg ctggttgctc ccattcttag gtgaatttaa ggaggccagg 5760
ctaaagccgt cgcatgtctg attgctcacc aggtaaatgt cgctaatgtt ttccaacgcg 5820
agaaggtgtt gagcgcggag ctgagtgacg tgacaacatg ggtatgccca attgccccat 5880
gttgggagga cgaaaatggt gacaagacag atggccagaa atacaccaac agcacgcatg 5940
atgtctactg gggatttatt ctttagtgcg gggaatacac cggcttttaa tacgattgag 6000
ggcgtctcct aacaagttac atcactcctg cccttcctca ccctcatctc catcacctcc 6060
ttcatctccg tcatctccgt catcaccctc cgcggcagcc ccttccacca taggtggaaa 6120
ccagggaggc aaatctactc catcgtcaaa gctgcacaca gtcaccctga tattgcaggt 6180
aggagcgggc tttgtcataa caaggtcctt aatcgcatcc ttcaaaacct cagcaaatat 6240
atgagtttgt aaaaagacca tgaaataaca gacaatggac tcccttagcg ggccaggttg 6300
tgggccgggt ccaggggcca ttccaaaggg gagacgactc aatggtgtaa gacgacattg 6360
tggaatagca agggcagttc ctcgccttag gttgtaaagg gaggtcttac tacctccata 6420
tacgaacaca ccggcgaccc aagttccttc gtcggtagtc ctttctacgt gactcctagc 6480
caggagagct cttaaacctt ctgcaatgtt ctcaaatttc gggttggaac ctccttgacc 6540
acgatgcttt ccaaaccacc ctcctttttt gcgcctgcct ccatcaccct gaccccgggg 6600
tccagtgctt gggccttctc ctgggtcatc tgcggggccc tgctctatcg ctcccggggg 6660
cacgtcaggc tcaccatctg ggccaccttc ttggtggtat tcaaaataat cggcttcccc 6720

tacagggtgg aaaaatggcc ttctacctgg agggggcctg cgcggtggag acccggatga 6780
tgatgactga ctactgggac tcctgggcct cttttctcca cgtccacgac ctctccccct 6840
ggctctttca cgacttcccc ccctggctct ttcacgtcct ctaccccggc ggcctccact 6900
acctcctcga ccccggcctc cactacctcc tcgaccccgg cctccactgc ctcctcgacc 6960
ccggcctcca cctcctgctc ctgcccctcc tgctcctgcc cctcctcctg ctcctgcccc 7020
tcctgcccct cctgctcctg cccctcctgc ccctcctgct cctgcccctc ctgcccctcc 7080
tgctcctgcc cctcctgccc ctcctcctgc tcctgcccct cctgcccctc ctcctgctcc 7140
tgcccctcct gcccctcctg ctcctgcccc tcctgcccct cctgctcctg cccctcctgc 7200
ccctcctgct cctgcccctc ctgctcctgc ccctcctgct cctgcccctc ctgctcctgc 7260
ccctcctgcc cctcctgccc ctcctcctgc tcctgcccct cctgctcctg cccctcctgc 7320
ccctcctgcc cctcctgctc ctgcccctcc tcctgctcct gcccctcctg cccctcctgc 7380
ccctcctcct gctcctgccc ctcctgcccc tcctcctgct cctgcccctc ctcctgctcc 7440
tgcccctcct gcccctcctg cccctcctcc tgctcctgcc cctcctgccc ctcctcctgc 7500
tcctgcccct cctcctgctc ctgcccctcc tgcccctcct gcccctcctc ctgctcctgc 7560
ccctcctcct gctcctgccc ctcctgcccc tcctgcccct cctgcccctc ctcctgctcc 7620
tgcccctcct cctgctcctg cccctcctgc tcctgcccct cccgctcctg ctcctgctcc 7680
tgttccaccg tgggtccctt tgcagccaat gcaacttgga cgtttttggg gtctccggac 7740
accatctcta tgtcttggcc ctgatcctga gccgcccggg gctcctggtc ttccgcctcc 7800
tcgtcctcgt cctcttcccc gtcctcgtcc atggttatca ccccctcttc tttgaggtcc 7860
actgccgccg gagccttctg gtccagatgt gtctcccttc tctcctaggc catttccagg 7920
tcctgtacct ggcccctcgt cagacatgat tcacactaaa agagatcaat agacatcttt 7980
attagacgac gctcagtgaa tacagggagt gcagactcct gccccctcca acagcccccc 8040
caccctcatc cccttcatgg tcgctgtcag acagatccag gtctgaaaat tccccatcct 8100
ccgaaccatc ctcgtcctca tcaccaatta ctcgcagccc ggaaaactcc cgctgaacat 8160
cctcaagatt tgcgtcctga gcctcaagcc aggcctcaaa ttcctcgtcc ccctttttgc 8220
tggacggtag ggatggggat tctcgggacc cctcctcttc ctcttcaagg tcaccagaca 8280
gagatgctac tggggcaacg gaagaaaagc tgggtgcggc ctgtgaggat cagcttatcg 8340
gtgaaccatc accctaatca agttttttgg ggtcgaggtg ccgtaaagca ctaaatcgga 8400
accctaaagg gagcccccga tttagagctt gacggggaaa gccggcgaac gtggcgagaa 8460
aggaagggaa gaaagcgaaa ggagcgggcg ctagggcgct ggcaagtgta gcggtcacgc 8520
tgcgcgtaac caccacaccc gccgcgctta atgcgccgct acagggcgcg tcaggtggca 8580
cttttcgggg aaatgtgcgc ggaaccccta tttgtttatt tttctaaata cattcaaata 8640
tgtatccgct catgagacaa taaccctgat aaatgcttca ataatattga aaaaggaaga 8700
gtcctgaggc ggaaagaacc agctgtggaa tgtgtgtcag ttagggtgtg gaaagtcccc 8760
aggctcccca gcaggcagaa gtatgcaaag catgcatctc aattagtcag caaccaggtg 8820
tggaaagtcc ccaggctccc cagcaggcag aagtatgcaa agcatgcatc tcaattagtc 8880
agcaaccata gtcccgcccc taactccgcc catcccgccc ctaactccgc ccagttccgc 8940
ccattctccg ccccatggct gactaatttt ttttatttat gcagaggccg aggccgcctc 9000
ggcctctgag ctattccaga agtagtgagg aggctttttt ggaggcctag gcttttgcaa 9060
agatcgatca agagacagga tgaggatcgt ttcgcatgat tgaacaagat ggattgcacg 9120
caggttctcc ggccgcttgg gtggagaggc tattcggcta tgactgggca caacagacaa 9180
tcggctgctc tgatgccgcc gtgttccggc tgtcagcgca ggggcgcccg gttctttttg 9240
tcaagaccga cctgtccggt gccctgaatg aactgcaaga cgaggcagcg cggctatcgt 9300
ggctggccac gacgggcgtt ccttgcgcag ctgtgctcga cgttgtcact gaagcgggaa 9360
gggactggct gctattgggc gaagtgccgg ggcaggatct cctgtcatct caccttgctc 9420
ctgccgagaa agtatccatc atggctgatg caatgcggcg gctgcatacg cttgatccgg 9480
ctacctgccc attcgaccac caagcgaaac atcgcatcga gcgagcacgt actcggatgg 9540
aagccggtct tgtcgatcag gatgatctgg acgaagagca tcaggggctc gcgccagccg 9600
aactgttcgc caggctcaag gcgagcatgc ccgacggcga ggatctcgtc gtgacccatg 9660
gcgatgcctg cttgccgaat atcatggtgg aaaatggccg cttttctgga ttcatcgact 9720
gtggccggct gggtgtggcg gaccgctatc aggacatagc gttggctacc cgtgatattg 9780
ctgaagagct tggcggcgaa tgggctgacc gcttcctcgt gctttacggt atcgccgctc 9840
ccgattcgca gcgcatcgcc ttctatcgcc ttcttgacga gttcttctga gcgggactct 9900
ggggttcgaa atgaccgacc aagcgacgcc caacctgcca tcacgagatt tcgattccac 9960
cgccgccttc tatgaaaggt tgggcttcgg aatcgttttc cgggacgccg gctggatgat 10020
cctccagcgc ggggatctca tgctggagtt cttcgcccac cctaggggga ggctaactga 10080
aacacggaag gagacaatac cggaaggaac ccgcgctatg acggcaataa aaagacagaa 10140
taaaacgcac ggtgttgggt cgtttgttca taaacgcggg gttcggtccc agggctggca 10200
ctctgtcgat accccaccga gaccccattg gggccaatac gcccgcgttt cttccttttc 10260
cccaccccac cccccaagtt cgggtgaagg cccagggctc gcagccaacg tcggggcggc 10320
aggccctgcc atagcctcag gttactcata tatactttag attgatttaa aacttcattt 10380

ttaatttaaa aggatctagg tgaagatcct ttttgataat ctcatgacca aaatccctta 10440
acgtgagttt tcgttccact gagcgtcaga ccccgtagaa aagatcaaag gatcttcttg 10500
agatcctttt tttctgcgcg taatctgctg cttgcaaaca aaaaaaccac cgctaccagc 10560
ggtggtttgt ttgccggatc aagagctacc aactcttttt ccgaaggtaa ctggcttcag 10620
cagagcgcag ataccaaata ctgtccttct agtgtagccg tagttaggcc accacttcaa 10680
gaactctgta gcaccgccta catacctcgc tctgctaatc ctgttaccag tggctgctgc 10740
cagtggcgat aagtcgtgtc ttaccgggtt ggactcaaga cgatagttac cggataaggc 10800
gcagcggtcg ggctgaacgg ggggttcgtg cacacagccc agcttggagc gaacgaccta 10860
caccgaactg agatacctac agcgtgagct atgagaaagc gccacgcttc ccgaagggag 10920
aaaggcggac aggtatccgg taagcggcag ggtcggaaca ggagagcgca cgagggagct 10980
tccaggggga aacgcctggt atctttatag tcctgtcggg tttcgccacc tctgacttga 11040
gcgtcgattt ttgtgatgct cgtcaggggg gcggagccta tggaaaaacg ccagcaacgc 11100
ggccttttta cggttcctgg ccttttgctg gccttttgct cacatgttct ttcctgcgtt 11160
atcccctgat tctgtggata accgtattac cgccatgcat 11200

<210> 5
<211> 12802
<212> DNA
<213> Künstliche Sequenz

<220>
<223> Beschreibung der künstlichen Sequenz: pIJO-71
      (EBN-GLUT-GFP)

<400> 5
   tagttattaa tagtaatcaa ttacggggtc attagttcat agcccatata tggagttccg 60
   cgttacataa cttacggtaa atggcccgcc tggctgaccg cccaacgacc cccgcccatt 120
   gacgtcaata atgacgtatg ttcccatagt aacgccaata gggactttcc attgacgtca 180
   atgggtggag tatttacggt aaactgccca cttggcagta catcaagtgt atcatatgcc 240
   aagtacgccc cctattgacg tcaatgacgg taaatggccc gcctggcatt atgcccagta 300
   catgacctta tgggactttc ctacttggca gtacatctac gtattagtca tcgctattac 360
   catggtgatg cggttttggc agtacatcaa tgggcgtgga tagcggtttg actcacgggg 420
   atttccaagt ctccacccca ttgacgtcaa tgggagtttg ttttggcacc aaaatcaacg 480
   ggactttcca aaatgtcgta acaactccgc cccattgacg caaatgggcg gtaggcgtgt 540
   acggtgggag gtctatataa gcagagctgg tttagtgaac cgtcagatcc gctagcgcta 600
   ccggactcag atctcgaggt cgacggtatc gataagcttc catggagccc agcagcaaga 660
   agctgacggg tcgcctcatg ctggccgtgg gaggagcagt gcttggctcc ctgcagtttg 720
   gctacaacac tggagtcatc aatgcccccc agaaggtgat cgaggagttc tacaaccaga 780
   catgggtcca ccgctatggg gagagcatcc tgcccaccac gctcaccacg ctctggtccc 840
   tctcagtggc catcttttct gttgggggca tgattggctc cttctctgtg ggccttttcg 900
   ttaaccgctt tggccggcgg aattcaatgc tgatgatgaa cctgctggcc ttcgtgtccg 960
   ccgtgctcat gggcttctcg aaactgggca agtcctttga gatgctgatc ctgggccgct 1020
   tcatcatcgg tgtgtactgc ggcctgacca caggcttcgt gcccatgtat gtgggtgaag 1080
   tgtcacccac agcccttcgt ggggccctgg gcaccctgca ccagctgggc atcgtcgtcg 1140
   gcatcctcat cgcccaggtg ttcggcctgg actccatcat gggcaacaag gacctgtggc 1200
   ccctgctgct gagcatcatc ttcatcccgg ccctgctgca gtgcatcgtg ctgcccttct 1260
   gccccgagag tccccgcttc ctgctcatca accgcaacga ggagaaccgg gccaagagtg 1320
   tgctaaagaa gctgcgcggg acagctgacg tgacccatga cctgcaggag atgaaggaag 1380
   agagtcggca gatgatgcgg gagaagaagg tcaccatcct ggagctgttc cgctcccccg 1440
   cctaccgcca gcccatcctc atcgctgtgg tgctgcagct gtcccagcag ctgtctggca 1500
   tcaacgctgt cttctattac tccacgagca tcttcgagaa ggcgggggtg cagcagcctg 1560
   tgtatgccac cattggctcc ggtatcgtca acacggcctt cactgtcgtg tcgctgtttg 1620
   tggtggagcg agcaggccgg cggaccctgc acctcatagg cctcgctggc atggcgggtt 1680
   gtgccatact catgaccatc gcgctagcac tgctggagca gctaccctgg atgtcctatc 1740
   tgagcatcgt ggccatcttt ggctttgtgg ccttctttga agtgggtcct ggccccatcc 1800
   catggttcat cgtggctgaa ctcttcagcc agggtccacg tccagctgcc attgccgttg 1860
   caggcttctc caactggacc tcaaatttca ttgtgggcat gtgcttccag tatgtggagc 1920
   aactgtgtgg tccctacgtc ttcatcatct tcactgtgct cctggttctg ttcttcatct 1980
   tcacctactt caaagttcct gagactaaag gccggacctt cgatgagatc gcttccggct 2040
   tccggcaggg gggagccagc caaagtgaca agacacccga ggagctgttc catcccctgg 2100
   gggctgattc ccaagtgtga ctcgagatgg ggatccgtaa tacgactcac tatagggaga 2160
   tctctgcagc cattcatctg atggccatca gatgaatggc tgcagtctag agattctata 2220
   gtgtcaccta aatgcaagct taaaacagct ctggggttgt acccacccca gaggcccacg 2280
   tggcggctag tactccggta ttgcggtacc cttgtacgcc tgttttatac tcccttcccg 2340
   taacttagac gcacaaaacc aagttcaata gaagggggta caaaccagta ccaccacgaa 2400
   caagcacttc tgtttccccg gtgatgtcgt atagactgct tgcgtggttg aaagcgacgg 2460
   atccgttatc cgcttatgta cttcgagaag cccagtacca cctcggaatc ttcgatgcgt 2520
   tgcgctcagc actcaacccc agagtgtagc ttaggctgat gagtctggac atccctcacc 2580
   ggtgacggtg gtccaggctg cgttggcggc ctacctatgg ctaacgccat gggacgctag 2640
   ttgtgaacaa ggtgtgaaga gcctattgag ctacataaga atcctccggc ccctgaatgc 2700
   ggctaatccc aacctcggag caggtggtca caaaccagtg attggcctgt cgtaacgcgc 2760
   aagtccgtgg cggaaccgac tactttgggt gtccgtgttt ccttttattt tattgtggct 2820
   gcttatggtg acaatcacag attgttatca taaagcgaat tggattgcgg ccgtcgacgc 2880
   ttgttctttt tgcagaagct cagaataaac gctcaactag cttgatatcg aattcctgca 2940
   gcccagcttc ctgcatgctg ctgctgctgc tgctgctggg cctgaggcta cagctctccc 3000
   tgggcatcat cccagttgag gaggagaacc cggacttctg gaaccgcgag gcagccgagg 3060
   ccctgggtgc cgccaagaag ctgcagcctg cacagacagc cgccaagaac ctcatcatct 3120
   tcctgggcga tgggatgggg gtgtctacgg tgacagctgc caggatccta aaagggcaga 3180
   agaaggacaa actggggcct gagatacccc tggccatgga ccgcttccca tatgtggctc 3240
   tgtccaagac atacaatgta gacaaacatg tgccagacag tggagccaca gccacggcct 3300
   acctgtgcgg ggtcaagggc aacttccaga ccattggctt gagtgcagcc gcccgcttta 3360
   accagtgcaa cacgacacgc ggcaacgagg tcatctccgt gatgaatcgg gccaagaaag 3420
   cagggaagtc agtgggagtg gtaaccacca cacgagtgca gcacgcctcg ccagccggca 3480
   cctacgccca cacggtgaac cgcaactggt actcggacgc cgacgtgcct gcctccgccc 3540
   gccaggaggg gtgccaggac atcgctacgc agctcatctc caacatggac attgacgtga 3600
   tcctaggtgg aggccgaaag tacatgtttc gcatgggaac cccagaccct gagtacccag 3660
   atgactacag ccaaggtggg accaggctgg acgggaagaa tctggtgcag gaatggctgg 3720
   cgaagcgcca gggtgcccgg tatgtgtgga accgcactga gctcatgcag gcttccctgg 3780
   acccgtctgt gacccatctc atgggtctct ttgagcctgg agacatgaaa tacgagatcc 3840
   accgagactc cacactggac ccctccctga tggagatgac agaggctgcc ctgcgcctgc 3900
   tgagcaggaa cccccgcggc ttcttcctct tcgtggaggg tggtcgcatc gaccatggtc 3960
   atcatgaaag cagggcttac cgggcactga ctgagacgat catgttcgac gacgccattg 4020
   agagggcggg ccagctcacc agcgaggagg acacgctgag cctcgtcact gccgaccact 4080
   cccacgtctt ctccttcgga ggctaccccc tgcgagggag ctccatcttc gggctggccc 4140
   ctggcaaggc ccgggacagg aaggcctaca cggtcctcct atacggaaac ggtccaggct 4200
   atgtgctcaa ggacggcgcc cggccggatg ttaccgagag cgagagcggg agccccgagt 4260
   atcggcagca gtcagcagtg cccctggacg aagagaccca cgcaggcgag gacgtggcgg 4320
   tgttcgcgcg cggcccgcag gcgcacctgg ttcacggcgt gcaggagcag accttcatag 4380
   cgcacgtcat ggccttcgcc gcctgcctgg agccctacac cgcctgcgac ctggcgcccc 4440
   ccgccggcac caccgacgcc gcgcacccgg gttaacccgt ggtccccgcg ttgcttcctc 4500
   tgctggccgg gacatcaggt ggcccccgct gaattggaat cgatcagaat tctgcagata 4560
   tccatcacac tggcggccgc gactctagat cataatcagc cataccacat ttgtagaggt 4620
   tttacttgct ttaaaaaacc tcccacacct ccccctgaac ctgaaacata aaatgaatgc 4680
   aattgttgtt gttaacttgt ttattgcagc ttataatggt tacaaataaa gcaatagcat 4740
   cacaaatttc acaaataaag catttttttc actgcattct agttgtggtt tgtccaaact 4800
   catcaatgta tcttaaggcg taaattgtaa gcgttaatat tttgttaaaa ttcgcgttaa 4860
   atttttgtta aatcagctca ttttttaacc aataggccga aatcggcaaa atcccttata 4920
   aatcaaaaga atagaccgag atagggttga gtgttgttcc agtttggaac aagagtccac 4980
   tattaaagaa cgtggactcc aacgtcaaag ggcgaaaaac cgtctatcag ggcgatggcc 5040
   cactcgactc tagagccccg ccgccggacg aactaaacct gactacggca tctctgcccc 5100
   ttcttcgctg gtacgaggag cgcttttgtt ttgtattcgg ggcagtgcat gtaatccctt 5160
   cagttggttg gtacaacttg ccaactgggc cctgttccac atgtgacacg gggggggacc 5220
   aaacacaaag gggttctctg actgtagttg acatccttat aaatggatgt gcacatttgc 5280
   caacactgag tggctttcat cctggagcag actttgcagt ctgtggactg caacacaaca 5340
   ttgcctttat gtgtaactct tggctgaagc tcttacacca atgctggggg acatgtacct 5400
   cccaggggcc caggaagact acgggaggct acaccaacgt caatcagagg ggcctgtgta 5460
   gctaccgata agcggaccct caagagggca ttagcaatag tgtttataag gcccccttgt 5520
   taaccctaaa cgggtagcat atgcttcccg ggtagtagta tatactatcc agactaaccc 5580
   taattcaata gcatatgtta cccaacggga agcatatgct atcgaattag ggttagtaaa 5640
   agggtcctaa ggaacagcga tatctcccac cccatgagct gtcacggttt tatttacatg 5700
   gggtcaggat tccacgaggg tagtgaacca ttttagtcac aagggcagtg gctgaagatc 5760
   aaggagcggg cagtgaactc tcctgaatct tcgcctgctt cttcattctc cttcgtttag 5820
   ctaatagaat aactgctgag ttgtgaacag taaggtgtat gtgaggtgct cgaaaacaag 5880
   gtttcaggtg acgcccccag aataaaattt ggacgggggg ttcagtggtg gcattgtgct 5940
   atgacaccaa tataaccctc acaaacccct tgggcaataa atactagtgt aggaatgaaa 6000
   cattctgaat atctttaaca atagaaatcc atggggtggg gacaagccgt aaagactgga 6060
   tgtccatctc acacgaattt atggctatgg gcaacacata atcctagtgc aatatgatac 6120
   tggggttatt aagatgtgtc ccaggcaggg accaagacag gtgaaccatg ttgttacact 6180
   ctatttgtaa caaggggaaa gagagtggac gccgacagca gcggactcca ctggttgtct 6240
   ctaacacccc cgaaaattaa acggggctcc acgccaatgg ggcccataaa caaagacaag 6300
   tggccactct tttttttgaa attgtggagt gggggcacgc gtcagccccc acacgccgcc 6360
   ctgcggtttt ggactgtaaa ataagggtgt aataacttgg ctgattgtaa ccccgctaac 6420
   cactgcggtc aaaccacttg cccacaaaac cactaatggc accccgggga atacctgcat 6480
   aagtaggtgg gcgggccaag ataggggcgc gattgctgcg atctggagga caaattacac 6540
   acacttgcgc ctgagcgcca agcacagggt tgttggtcct catattcacg aggtcgctga 6600
   gagcacggtg ggctaatgtt gccatgggta gcatatacta cccaaatatc tggatagcat 6660
   atgctatcct aatctatatc tgggtagcat aggctatcct aatctatatc tgggtagcat 6720
   atgctatcct aatctatatc tgggtagtat atgctatcct aatttatatc tgggtagcat 6780
   aggctatcct aatctatatc tgggtagcat atgctatcct aatctatatc tgggtagtat 6840
   atgctatcct aatctgtatc cgggtagcat atgctatcct aatagagatt agggtagtat 6900
   atgctatcct aatttatatc tgggtagcat atactaccca aatatctgga tagcatatgc 6960
   tatcctaatc tatatctggg tagcatatgc tatcctaatc tatatctggg tagcataggc 7020
   tatcctaatc tatatctggg tagcatatgc tatcctaatc tatatctggg tagtatatgc 7080
   tatcctaatt tatatctggg tagcataggc tatcctaatc tatatctggg tagcatatgc 7140
   tatcctaatc tatatctggg tagtatatgc tatcctaatc tgtatccggg tagcatatgc 7200
   tatcctcatg catatacagt cagcatatga tacccagtag tagagtggga gtgctatcct 7260
   ttgcatatgc cgccacctcc caagggggcg tgaattttcg ctgcttgtcc ttttcctgca 7320
   tgctggttgc tcccattctt aggtgaattt aaggaggcca ggctaaagcc gtcgcatgtc 7380
   tgattgctca ccaggtaaat gtcgctaatg ttttccaacg cgagaaggtg ttgagcgcgg 7440
   agctgagtga cgtgacaaca tgggtatgcc caattgcccc atgttgggag gacgaaaatg 7500
   gtgacaagac agatggccag aaatacacca acagcacgca tgatgtctac tggggattta 7560
   ttctttagtg cgggggaata cacggctttt aatacgattg agggcgtctc ctaacaagtt 7620
   acatcactcc tgcccttcct caccctcatc tccatcacct ccttcatctc cgtcatctcc 7680
   gtcatcaccc tccgcggcag ccccttccac cataggtgga aaccagggag gcaaatctac 7740
   tccatcgtca aagctgcaca cagtcaccct gatattgcag gtaggagcgg gctttgtcat 7800
   aacaaggtcc ttaatcgcat ccttcaaaac ctcagcaaat atatgagttt gtaaaaagac 7860
   catgaaataa cagacaatgg actcccttag cgggccaggt tgtgggccgg gtccaggggc 7920
   cattccaaag gggagacgac tcaatggtgt aagacgacat tgtggaatag caagggcagt 7980
   tcctcgcctt aggttgtaaa gggaggtctt actacctcca tatacgaaca caccggcgac 8040
   ccaagttcct tcgtcggtag tcctttctac gtgactccta gccaggagag ctcttaaacc 8100
   ttctgcaatg ttctcaaatt tcgggttgga acctccttga ccacgatgct ttccaaacca 8160
   ccctcctttt ttgcgcctgc ctccatcacc ctgaccccgg ggtccagtgc ttgggccttc 8220
   tcctgggtca tctgcggggc cctgctctat cgctcccggg ggcacgtcag gctcaccatc 8280
   tgggccacct tcttggtggt attcaaaata atcggcttcc cctacagggt ggaaaaatgg 8340
   ccttctacct ggagggggcc tgcgcggtgg agacccggat gatgatgact gactactggg 8400
   actcctgggc ctcttttctc cacgtccacg acctctcccc ctggctcttt cacgacttcc 8460
   ccccctggct ctttcacgtc ctctaccccg gcggcctcca ctacctcctc gaccccggcc 8520
   tccactacct cctcgacccc ggcctccact gcctcctcga ccccggcctc cacctcctgc 8580
   tcctgcccct cctgctcctg cccctcctcc tgctcctgcc cctcctgccc ctcctgctcc 8640
   tgcccctcct gcccctcctg ctcctgcccc tcctgcccct cctgctcctg cccctcctgc 8700
   ccctcctcct gctcctgccc ctcctgcccc tcctcctgct cctgcccctc ctgcccctcc 8760
   tgctcctgcc cctcctgccc ctcctgctcc tgcccctcct gcccctcctg ctcctgcccc 8820
   tcctgctcct gcccctcctg ctcctgcccc tcctgctcct gcccctcctg cccctcctgc 8880
   ccctcctcct gctcctgccc ctcctgctcc tgcccctcct gcccctcctg cccctcctgc 8940
   tcctgcccct cctcctgctc ctgcccctcc tgcccctcct gcccctcctc ctgctcctgc 9000
   ccctcctgcc cctcctcctg ctcctgcccc tcctcctgct cctgcccctc ctgcccctcc 9060
   tgcccctcct cctgctcctg cccctcctgc ccctcctcct gctcctgccc ctcctcctgc 9120
   tcctgcccct cctgcccctc ctgcccctcc tcctgctcct gcccctcctc ctgctcctgc 9180
   ccctcctgcc cctcctgccc ctcctgcccc tcctcctgct cctgcccctc ctcctgctcc 9240
   tgcccctcct gctcctgccc ctcccgctcc tgctcctgct cctgttccac cgtgggtccc 9300
   tttgcagcca atgcaacttg gacgtttttg gggtctccgg acaccatctc tatgtcttgg 9360
   ccctgatcct gagccgcccg gggctcctgg tcttccgcct cctcgtcctc gtcctcttcc 9420
   ccgtcctcgt ccatggttat caccccctct tctttgaggt ccactgccgc cggagccttc 9480
   tggtccagat gtgtctccct tctctcctag gccatttcca ggtcctgtac ctggcccctc 9540
   gtcagacatg attcacacta aaagagatca atagacatct ttattagacg acgctcagtg 9600
   aatacaggga gtgcagactc ctgccccctc caacagcccc cccaccctca tccccttcat 9660
   ggtcgctgtc agacagatcc aggtctgaaa attccccatc ctccgaacca tcctcgtcct 9720
   catcaccaat tactcgcagc ccggaaaact cccgctgaac atcctcaaga tttgcgtcct 9780
   gagcctcaag ccaggcctca aattcctcgt cccccttttt gctggacggt agggatgggg 9840
   attctcggga cccctcctct tcctcttcaa ggtcaccaga cagagatgct actggggcaa 9900
   cggaagaaaa gctgggtgcg gcctgtgagg atcagcttat cggtgaacca tcaccctaat 9960
   caagtttttt ggggtcgagg tgccgtaaag cactaaatcg gaaccctaaa gggagccccc 10020
   gatttagagc ttgacgggga aagccggcga acgtggcgag aaaggaaggg aagaaagcga 10080
   aaggagcggg cgctagggcg ctggcaagtg tagcggtcac gctgcgcgta accaccacac 10140
   ccgccgcgct taatgcgccg ctacagggcg cgtcaggtgg cacttttcgg ggaaatgtgc 10200
   gcggaacccc tatttgttta tttttctaaa tacattcaaa tatgtatccg ctcatgagac 10260
   aataaccctg ataaatgctt caataatatt gaaaaaggaa gagtcctgag gcggaaagaa 10320
   ccagctgtgg aatgtgtgtc agttagggtg tggaaagtcc ccaggctccc cagcaggcag 10380
   aagtatgcaa agcatgcatc tcaattagtc agcaaccagg tgtggaaagt ccccaggctc 10440
   cccagcaggc agaagtatgc aaagcatgca tctcaattag tcagcaacca tagtcccgcc 10500
   cctaactccg cccatcccgc ccctaactcc gcccagttcc gcccattctc cgccccatgg 10560
   ctgactaatt ttttttattt atgcagaggc cgaggccgcc tcggcctctg agctattcca 10620
   gaagtagtga ggaggctttt ttggaggcct aggcttttgc aaagatcgat caagagacag 10680
   gatgaggatc gtttcgcatg attgaacaag atggattgca cgcaggttct ccggccgctt 10740
   gggtggagag gctattcggc tatgactggg cacaacagac aatcggctgc tctgatgccg 10800
   ccgtgttccg gctgtcagcg caggggcgcc cggttctttt tgtcaagacc gacctgtccg 10860
   gtgccctgaa tgaactgcaa gacgaggcag cgcggctatc gtggctggcc acgacgggcg 10920
   ttccttgcgc agctgtgctc gacgttgtca ctgaagcggg aagggactgg ctgctattgg 10980
   gcgaagtgcc ggggcaggat ctcctgtcat ctcaccttgc tcctgccgag aaagtatcca 11040
   tcatggctga tgcaatgcgg cggctgcata cgcttgatcc ggctacctgc ccattcgacc 11100
   accaagcgaa acatcgcatc gagcgagcac gtactcggat ggaagccggt cttgtcgatc 11160
   aggatgatct ggacgaagag catcaggggc tcgcgccagc cgaactgttc gccaggctca 11220
   aggcgagcat gcccgacggc gaggatctcg tcgtgaccca tggcgatgcc tgcttgccga 11280
   atatcatggt ggaaaatggc cgcttttctg gattcatcga ctgtggccgg ctgggtgtgg 11340
   cggaccgcta tcaggacata gcgttggcta cccgtgatat tgctgaagag cttggcggcg 11400
   aatgggctga ccgcttcctc gtgctttacg gtatcgccgc tcccgattcg cagcgcatcg 11460
   ccttctatcg ccttcttgac gagttcttct gagcgggact ctggggttcg aaatgaccga 11520
   ccaagcgacg cccaacctgc catcacgaga tttcgattcc accgccgcct tctatgaaag 11580
   gttgggcttc ggaatcgttt tccgggacgc cggctggatg atcctccagc gcggggatct 11640
   catgctggag ttcttcgccc accctagggg gaggctaact gaaacacgga aggagacaat 11700
   accggaagga acccgcgcta tgacggcaat aaaaagacag aataaaacgc acggtgttgg 11760
   gtcgtttgtt cataaacgcg gggttcggtc ccagggctgg cactctgtcg ataccccacc 11820
   gagaccccat tggggccaat acgcccgcgt ttcttccttt tccccacccc accccccaag 11880
   ttcgggtgaa ggcccagggc tcgcagccaa cgtcggggcg gcaggccctg ccatagcctc 11940
   aggttactca tatatacttt agattgattt aaaacttcat ttttaattta aaaggatcta 12000
   ggtgaagatc ctttttgata atctcatgac caaaatccct taacgtgagt tttcgttcca 12060
   ctgagcgtca gaccccgtag aaaagatcaa aggatcttct tgagatcctt tttttctgcg 12120
   cgtaatctgc tgcttgcaaa caaaaaaacc accgctacca gcggtggttt gtttgccgga 12180
   tcaagagcta ccaactcttt ttccgaaggt aactggcttc agcagagcgc agataccaaa 12240
   tactgtcctt ctagtgtagc cgtagttagg ccaccacttc aagaactctg tagcaccgcc 12300
   tacatacctc gctctgctaa tcctgttacc agtggctgct gccagtggcg ataagtcgtg 12360
   tcttaccggg ttggactcaa gacgatagtt accggataag gcgcagcggt cgggctgaac 12420
   ggggggttcg tgcacacagc ccagcttgga gcgaacgacc tacaccgaac tgagatacct 12480
   acagcgtgag ctatgagaaa gcgccacgct tcccgaaggg agaaaggcgg acaggtatcc 12540
   ggtaagcggc agggtcggaa caggagagcg cacgagggag cttccagggg gaaacgcctg 12600
   gtatctttat agtcctgtcg ggtttcgcca cctctgactt gagcgtcgat ttttgtgatg 12660
   ctcgtcaggg gggcggagcc tatggaaaaa cgccagcaac gcggcctttt tacggttcct 12720
   ggccttttgc tggccttttg ctcacatgtt ctttcctgcg ttatcccctg attctgtgga 12780
   taaccgtatt accgccatgc at 12802

<210> 6
<211> 21

<212> DNA
<213> Künstliche Sequenz

<220>
<223> Beschreibung der künstlichen Sequenz: Primer i100

<400> 6
ataagcttcc atggagccca g                                                21

<210> 7
<211> 24
<212> DNA
<213> Künstliche Sequenz

<220>
<223> Beschreibung der künstlichen Sequenz: Primer i101

<400> 7
atctcgagtc acacttggga atca                                             24

<210> 8
<211> 25
<212> DNA
<213> Künstliche Sequenz

<220>
<223> Beschreibung der künstlichen Sequenz: Primer i102

<400> 8
ataagcttcc atggacagta gcacc                                            25

<210> 9
<211> 26
<212> DNA
<213> Künstliche Sequenz

<220>
<223> Beschreibung der künstlichen Sequenz: Primer i103

<400> 9
atctcgagtc aggcaaaata tgcatg                                           26

<210> 10
<211> 19
<212> DNA
<213> Künstliche Sequenz

<220>
<223> Beschreibung der künstlichen Sequenz: Primer i104

<400> 10
gagcaggaga ccaaacgac                                                   19

<210> 11
<211> 19
<212> DNA

<213> Künstliche Sequenz

<220>
<223> Beschreibung der künstlichen Sequenz: Primer i105

<400> 11
gttgggtagg aagagatgg                                                19


<210> 12
<211> 4927
<212> DNA
<213> Künstliche Sequenz

<220>
<223> Beschreibung der künstlichen Sequenz: pIJO-62 (GLU
      PRF)

<400> 12
agcgcccaat acgcaaaccg cctctccccg cgcgttggcc gattcattaa tgcagctggc 60
acgacaggtt tcccgactgg aaagcgggca gtgagcgcaa cgcaattaat gtgagttagc 120
tcactcatta ggcaccccag gctttacact ttatgcttcc ggctcgtatg ttgtgtggaa 180
ttgtgagcgg ataacaattt cacacaggaa acagctatga ccatgattac gccaagcttg 240
catttaggtg acactataga atctctagac tgcagccatt catctgatgg ccatcagatg 300
aatggctgca gagatctccc tatagtgagt cgtattacgg atccccatct cgagtcacac 360
ttgggaatca gcccccaggg gatggaacag ctcctcgggt gtcttgtcac tttggctggc 420
tcccccctgc cggaagccgg aagcgatctc atcgaaggtc cggcctttag tctcaggaac 480
tttgaagtag gtgaagatga agaacagaac caggagcaca gtgaagatga tgaagacgta 540
gggaccacac agttgctcca catactggaa gcacatgccc acaatgaaat ttgaggtcca 600
gttggagaag cctgcaacgg caatggcagc tggacgtgga ccctggctga agagttcagc 660
cacgatgaac catgggatgg ggccaggacc cacttcaaag aaggccacaa agccaaagat 720
ggccacgatg ctcagatagg acatccaggg tagctgctcc agcagtgcta gcgcgatggt 780
catgagtatg gcacaacccg ccatgccagc gaggcctatg aggtgcaggg tccgccggcc 840
tgctcgctcc accacaaaca gcgacacgac agtgaaggcc gtgttgacga taccggagcc 900
aatggtggca tacacaggct gctgcacccc cgccttctcg aagatgctcg tggagtaata 960
gaagacagcg ttgatgccag acagctgctg ggacagctgc agcaccacag cgatgaggat 1020
gggctggcgg taggcggggg agcggaacag ctccaggatg gtgaccttct tctcccgcat 1080
catctgccga ctctcttcct tcatctcctg caggtcatgg gtcacgtcag ctgtcccgcg 1140
cagcttcttt agcacactct tggcccggtt ctcctcgttg cggttgatga gcaggaagcg 1200
gggactctcg gggcagaagg gcagcacgat gcactgcagc agggccggga tgaagatgat 1260
gctcagcagc aggggccaca ggtccttgtt gcccatgatg gagtccaggc cgaacacctg 1320
ggcgatgagg atgccgacga cgatgcccag ctggtgcagg gtgcccaggg ccccacgaag 1380
ggctgtgggt gacacttcac ccacatacat gggcacgaag cctgtggtca ggccgcagta 1440
cacaccgatg atgaagcggc ccaggatcag catctcaaag gacttgccca gtttcgagaa 1500
gcccatgagc acggcggaca cgaaggccag caggttcatc atcagcattg aattccgccg 1560
gccaaagcgg ttaacgaaaa ggcccacaga gaaggagcca atcatgcccc caacagaaaa 1620
gatggccact gagagggacc agagcgtggt gagcgtggtg ggcaggatgc tctccccata 1680
gcggtggacc catgtctggt tgtagaactc ctcgatcacc ttctgggggg cattgatgac 1740
tccagtgttg tagccaaact gcagggagcc aagcactgct cctcccacgg ccagcatgag 1800
gcgacccgtc agcttcttgc tgctgggctc catggaagct tatgggtacc ggtcgccacc 1860
atggtgagca agggcgagga gctgttcacc ggggtggtgc ccatcctggt cgagctggac 1920
ggcgacgtaa acggccacaa gttcagcgtg tccggcgagg gcgagggcga tgccacctac 1980
ggcaagctga ccctgaagtt catctgcacc accggcaagc tgcccgtgcc ctggcccacc 2040
ctcgtgacca ccctgaccta cggcgtgcag tgcttcagcc gctaccccga ccacatgaag 2100
cagcacgact tcttcaagtc cgccatgccc gaaggctacg tccaggagcg caccatcttc 2160
ttcaaggacg acggcaacta caagacccgc gccgaggtga agttcgaggg cgacaccctg 2220
gtgaaccgca tcgagctgaa gggcatcgac ttcaaggagg acggcaacat cctggggcac 2280
aagctggagt acaactacaa cagccacaac gtctatatca tggccgacaa gcagaagaac 2340
ggcatcaagg cgaatttcaa gatccgccac aacatcgagg acggcagcgt gcagctcgcc 2400
gaccactacc agcagaacac ccccatcggc gacggccccg tgctgctgcc cgacaaccac 2460
tacctgagca cccagtccgc cctgagcaaa gaccccaacg agaagcgcga tcacatggtc 2520

ctgctggagt tcgtgaccgc cgccgggatc actctcggca tggacgagct gtacaagtaa 2580
agcggccgcg actctagaat tccaactgag cgccggtcgc taccattacc aacttgtctg 2640
gtgtcaaaaa taataggcct actagtcggc cgtacgggcc ctttcgtctc gcgcgtttcg 2700
gtgatgacgg tgaaaacctc tgacacatgc agctcccgga gacggtcaca gcttgtctgt 2760
aagcggatgc cgggagcaga caagcccgtc agggcgcgtc agcgggtgtt ggcgggtgtc 2820
ggggctggct taactatgcg gcatcagagc agattgtact gagagtgcac catatgcggt 2880
gtgaaatacc gcacagatgc gtaaggagaa aataccgcat caggcggcct taagggcctc 2940
gtgatacgcc tatttttata ggttaatgtc atgataataa tggtttctta gacgtcaggt 3000
ggcacttttc ggggaaatgt gcgcggaacc cctatttgtt tatttttcta aatacattca 3060
aatatgtatc cgctcatgag acaataaccc tgataaatgc ttcaataata ttgaaaaagg 3120
aagagtatga gtattcaaca tttccgtgtc gcccttattc ccttttttgc ggcattttgc 3180
cttcctgttt ttgctcaccc agaaacgctg gtgaaagtaa aagatgctga agatcagttg 3240
ggtgcacgag tgggttacat cgaactggat ctcaacagcg gtaagatcct tgagagtttt 3300
cgccccgaag aacgttttcc aatgatgagc acttttaaag ttctgctatg tggcgcggta 3360
ttatcccgta ttgacgccgg gcaagagcaa ctcggtcgcc gcatacacta ttctcagaat 3420
gacttggttg agtactcacc agtcacagaa aagcatctta cggatggcat gacagtaaga 3480
gaattatgca gtgctgccat aaccatgagt gataacactg cggccaactt acttctgaca 3540
acgatcggag gaccgaagga gctaaccgct tttttgcaca acatggggga tcatgtaact 3600
cgccttgatc gttgggaacc ggagctgaat gaagccatac caaacgacga gcgtgacacc 3660
acgatgcctg tagcaatggc aacaacgttg cgcaaactat taactggcga actacttact 3720
ctagcttccc ggcaacaatt aatagactgg atggaggcgg ataaagttgc aggaccactt 3780
ctgcgctcgg cccttccggc tggctggttt attgctgata aatctggagc cggtgagcgt 3840
gggtctcgcg gtatcattgc agcactgggg ccagatggta agccctcccg tatcgtagtt 3900
atctacacga cggggagtca ggcaactatg gatgaacgaa atagacagat cgctgagata 3960
ggtgcctcac tgattaagca ttggtaactg tcagaccaag tttactcata tatactttag 4020
attgatttaa aacttcattt ttaatttaaa aggatctagg tgaagatcct ttttgataat 4080
ctcatgacca aaatccctta acgtgagttt tcgttccact gagcgtcaga ccccgtagaa 4140
aagatcaaag gatcttcttg agatcctttt tttctgcgcg taatctgctg cttgcaaaca 4200
aaaaaaccac cgctaccagc ggtggtttgt ttgccggatc aagagctacc aactcttttt 4260
ccgaaggtaa ctggcttcag cagagcgcag ataccaaata ctgtccttct agtgtagccg 4320
tagttaggcc accacttcaa gaactctgta gcaccgccta catacctcgc tctgctaatc 4380
ctgttaccag tggctgctgc cagtggcgat aagtcgtgtc ttaccgggtt ggactcaaga 4440
cgatagttac cggataaggc gcagcggtcg ggctgaacgg ggggttcgtg cacacagccc 4500
agcttggagc gaacgaccta caccgaactg agatacctac agcgtgagct atgagaaagc 4560
gccacgcttc ccgaagggag aaaggcggac aggtatccgg taagcggcag ggtcggaaca 4620
ggagagcgca cgagggagct tccaggggga aacgcctggt atctttatag tcctgtcggg 4680
tttcgccacc tctgacttga gcgtcgattt ttgtgatgct cgtcaggggg gcggagccta 4740
tggaaaaacg ccagcaacgc ggccttttta cggttcctgg ccttttgctg gccttttgct 4800
cacatgttct ttcctgcgtt atcccctgat tctgtggata accgtattac cgcctttgag 4860
tgagctgata ccgctcgccg cagccgaacg accgagcgca gcgagtcagt gagcgaggaa 4920
gcggaag 4927

<210> 13
<211> 7000
<212> DNA
<213> Künstliche Sequenz

<220>
<223> Beschreibung der künstlichen Sequenz: pIJO-53 (SEAP
cD3)

<400> 13
gacggatcgg gagatctccc gatcccctat ggtcgactct cagtacaatc tgctctgatg 60
ccgcatagtt aagccagtat ctgctccctg cttgtgtgtt ggaggtcgct gagtagtgcg 120
cgagcaaaat ttaagctaca acaaggcaag gcttgaccga caattgcatg aagaatctgc 180
ttagggttag gcgttttgcg ctgcttcgcg atgtacgggc cagatatacg cgttgacatt 240
gattattgac tagttattaa tagtaatcaa ttacggggtc attagttcat agcccatata 300
tggagttccg cgttacataa cttacggtaa atggcccgcc tggctgaccg cccaacgacc 360
cccgcccatt gacgtcaata atgacgtatg ttcccatagt aacgccaata gggactttcc 420
attgacgtca atgggtggac tatttacggt aaactgccca cttggcagta catcaagtgt 480

atcatatgcc aagtacgccc cctattgacg tcaatgacgg taaatggccc gcctggcatt 540
atgcccagta catgacctta tgggactttc ctacttggca gtacatctac gtattagtca 600
tcgctattac catggtgatg cggttttggc agtacatcaa tgggcgtgga tagcggtttg 660
actcacgggg atttccaagt ctccacccca ttgacgtcaa tgggagtttg ttttggcacc 720
aaaatcaacg ggactttcca aaatgtcgta acaactccgc cccattgacg caaatgggcg 780
gtaggcgtgt acggtgggag gtctatataa gcagagctct ctggctaact agagaaccca 840
ctgcttactg gcttatcgaa attaatacga ctcactatag ggagacccaa gcttcctgca 900
tgctgctgct gctgctgctg ctgggcctga ggctacagct ctccctgggc atcatcccag 960
ttgaggagga gaacccggac ttctggaacc gcgaggcagc cgaggccctg ggtgccgcca 1020
agaagctgca gcctgcacag acagccgcca agaacctcat catcttcctg ggcgatggga 1080
tgggggtgtc tacggtgaca gctgccagga tcctaaaagg gcagaagaag gacaaactgg 1140
ggcctgagat acccctggcc atggaccgct tcccatatgt ggctctgtcc aagacataca 1200
atgtagacaa acatgtgcca gacagtggag ccacagccac ggcctacctg tgcggggtca 1260
agggcaactt ccagaccatt ggcttgagtg cagccgcccg ctttaaccag tgcaacacga 1320
cacgcggcaa cgaggtcatc tccgtgatga atcgggccaa gaaagcaggg aagtcagtgg 1380
gagtggtaac caccacacga gtgcagcacg cctcgccagc cggcacctac gcccacacgg 1440
tgaaccgcaa ctggtactcg gacgccgacg tgcctgcctc cgcccgccag gaggggtgcc 1500
aggacatcgc tacgcagctc atctccaaca tggacattga cgtgatccta ggtggaggcc 1560
gaaagtacat gtttcgcatg ggaaccccag accctgagta cccagatgac tacagccaag 1620
gtgggaccag gctggacggg aagaatctgg tgcaggaatg gctggcgaag cgccagggtg 1680
cccggtatgt gtggaaccgc actgagctca tgcaggcttc cctggacccg tctgtgaccc 1740
atctcatggg tctctttgag cctggagaca tgaaatacga gatccaccga gactccacac 1800
tggacccctc cctgatggag atgacagagg ctgccctgcg cctgctgagc aggaaccccc 1860
gcggcttctt cctcttcgtg gagggtggtc gcatcgacca tggtcatcat gaaagcaggg 1920
cttaccgggc actgactgag acgatcatgt tcgacgacgc cattgagagg gcgggccagc 1980
tcaccagcga ggaggacacg ctgagcctcg tcactgccga ccactcccac gtcttctcct 2040
tcggaggcta ccccctgcga gggagctcca tcttcgggct ggcccctggc aaggcccggg 2100
acaggaaggc ctacacggtc ctcctatacg gaaacggtcc aggctatgtg ctcaaggacg 2160
gcgcccggcc ggatgttacc gagagcgaga gcgggagccc cgagtatcgg cagcagtcag 2220
cagtgcccct ggacgaagag acccacgcag gcgaggacgt ggcggtgttc gcgcgcggcc 2280
cgcaggcgca cctggttcac ggcgtgcagg agcagacctt catagcgcac gtcatggcct 2340
tcgccgcctg cctggagccc tacaccgcct gcgacctggc gccccccgcc ggcaccaccg 2400
acgccgcgca cccgggttaa cccgtggtcc ccgcgttgct tcctctgctg gccgggacat 2460
caggtggccc ccgctgaatt ggaatcgatc agaattctgc agatatccat cacactggcg 2520
gccgctcgag catgcatcta gagggcccta ttctatagtg tcacctaaat gctagagctc 2580
gctgatcagc ctcgactgtg ccttctagtt gccagccatc tgttgtttgc ccctcccccg 2640
tgccttcctt gaccctggaa ggtgccactc ccactgtcct ttcctaataa aatgaggaaa 2700
ttgcatcgca ttgtctgagt aggtgtcatt ctattctggg gggtggggtg gggcaggaca 2760
gcaaggggga ggattgggaa gacaatagca ggcatgctgg ggatgcggtg ggctctatgg 2820
cttctgaggc ggaaagaacc agctggggct ctagggggta tccccacgcg ccctgtagcg 2880
gcgcattaag cgcggcgggt gtggtggtta cgcgcagcgt gaccgctaca cttgccagcg 2940
ccctagcgcc cgctcctttc gctttcttcc cttcctttct cgccacgttc gccggctttc 3000
cccgtcaagc tctaaatcgg ggcatccctt tagggttccg atttagtgct ttacggcacc 3060
tcgaccccaa aaaacttgat tagggtgatg gttcacgtag tgggccatcg ccctgataga 3120
cggtttttcg ccctttgacg ttggagtcca cgttctttaa tagtggactc ttgttccaaa 3180
ctggaacaac actcaaccct atctcggtct attcttttga tttataaggg attttgggga 3240
tttcggccta ttggttaaaa aatgagctga tttaacaaaa atttaacgcg aattaattct 3300
gtggaatgtg tgtcagttag ggtgtggaaa gtccccaggc tccccaggca ggcagaagta 3360
tgcaaagcat gcatctcaat tagtcagcaa ccaggtgtgg aaagtcccca ggctccccag 3420
caggcagaag tatgcaaagc atgcatctca attagtcagc aaccatagtc ccgcccctaa 3480
ctccgcccat cccgccccta actccgccca gttccgccca ttctccgccc catggctgac 3540
taattttttt tatttatgca gaggccgagg ccgcctctgc ctctgagcta ttccagaagt 3600
agtgaggagg cttttttgga ggcctaggct tttgcaaaaa gctcccggga gcttgtatat 3660
ccattttcgg atctgatcaa gagacaggat gaggatcgtt tcgcatgatt gaacaagatg 3720
gattgcacgc aggttctccg gccgcttggg tggagaggct attcggctat gactgggcac 3780
aacagacaat cggctgctct gatgccgccg tgttccggct gtcagcgcag gggcgcccgg 3840
ttctttttgt caagaccgac ctgtccggtg ccctgaatga actgcaggac gaggcagcgc 3900
ggctatcgtg gctggccacg acgggcgttc cttgcgcagc tgtgctcgac gttgtcactg 3960
aagcgggaag ggactggctg ctattgggcg aagtgccggg gcaggatctc ctgtcatctc 4020
accttgctcc tgccgagaaa gtatccatca tggctgatgc aatgcggcgg ctgcatacgc 4080
ttgatccggc tacctgccca ttcgaccacc aagcgaaaca tcgcatcgag cgagcacgta 4140

ctcggatgga agccggtctt gtcgatcagg atgatctgga cgaagagcat caggggctcg 4200
cgccagccga actgttcgcc aggctcaagg cgcgcatgcc cgacggcgag gatctcgtcg 4260
tgacccatgg cgatgcctgc ttgccgaata tcatggtgga aaatggccgc ttttctggat 4320
tcatcgactg tggccggctg ggtgtggcgg accgctatca ggacatagcg ttggctaccc 4380
gtgatattgc tgaagagctt ggcggcgaat gggctgaccg cttcctcgtg ctttacggta 4440
tcgccgctcc cgattcgcag cgcatcgcct tctatcgcct tcttgacgag ttcttctgag 4500
cgggactctg gggttcgaaa tgaccgacca agcgacgccc aacctgccat cacgagattt 4560
cgattccacc gccgccttct atgaaaggtt gggcttcgga atcgttttcc gggacgccgg 4620
ctggatgatc ctccagcgcg gggatctcat gctggagttc ttcgcccacc ccaacttgtt 4680
tattgcagct tataatggtt acaaataaag caatagcatc acaaatttca caaataaagc 4740
atttttttca ctgcattcta gttgtggttt gtccaaactc atcaatgtat cttatcatgt 4800
ctgtataccg tcgacctcta gctagagctt ggcgtaatca tggtcatagc tgtttcctgt 4860
gtgaaattgt tatccgctca caattccaca caacatacga gccggaagca taaagtgtaa 4920
agcctggggt gcctaatgag tgagctaact cacattaatt gcgttgcgct cactgcccgc 4980
tttccagtcg ggaaacctgt cgtgccagct gcattaatga atcggccaac gcgcggggag 5040
aggcggtttg cgtattgggc gctcttccgc ttcctcgctc actgactcgc tgcgctcggt 5100
cgttcggctg cggcgagcgg tatcagctca ctcaaaggcg gtaatacggt tatccacaga 5160
atcaggggat aacgcaggaa agaacatgtg agcaaaaggc cagcaaaagg ccaggaaccg 5220
taaaaaggcc gcgttgctgg cgtttttcca taggctccgc ccccctgacg agcatcacaa 5280
aaatcgacgc tcaagtcaga ggtggcgaaa cccgacagga ctataaagat accaggcgtt 5340
tccccctgga agctccctcg tgcgctctcc tgttccgacc ctgccgctta ccggatacct 5400
gtccgccttt ctcccttcgg gaagcgtggc gctttctcaa tgctcacgct gtaggtatct 5460
cagttcggtg taggtcgttc gctccaagct gggctgtgtg cacgaacccc ccgttcagcc 5520
cgaccgctgc gccttatccg gtaactatcg tcttgagtcc aacccggtaa gacacgactt 5580
atcgccactg gcagcagcca ctggtaacag gattagcaga gcgaggtatg taggcggtgc 5640
tacagagttc ttgaagtggt ggcctaacta cggctacact agaaggacag tatttggtat 5700
ctgcgctctg ctgaagccag ttaccttcgg aaaaagagtt ggtagctctt gatccggcaa 5760
acaaaccacc gctggtagcg gtggtttttt tgtttgcaag cagcagatta cgcgcagaaa 5820
aaaaggatct caagaagatc ctttgatctt ttctacgggg tctgacgctc agtggaacga 5880
aaactcacgt taagggattt tggtcatgag attatcaaaa aggatcttca cctagatcct 5940
tttaaattaa aaatgaagtt ttaaatcaat ctaaagtata tatgagtaaa cttggtctga 6000
cagttaccaa tgcttaatca gtgaggcacc tatctcagcg atctgtctat ttcgttcatc 6060
catagttgcc tgactccccg tcgtgtagat aactacgata cgggagggct taccatctgg 6120
ccccagtgct gcaatgatac cgcgagaccc acgctcaccg gctccagatt tatcagcaat 6180
aaaccagcca gccggaaggg ccgagcgcag aagtggtcct gcaactttat ccgcctccat 6240
ccagtctatt aattgttgcc gggaagctag agtaagtagt tcgccagtta atagtttgcg 6300
caacgttgtt gccattgcta caggcatcgt ggtgtcacgc tcgtcgtttg gtatggcttc 6360
attcagctcc ggttcccaac gatcaaggcg agttacatga tcccccatgt tgtgcaaaaa 6420
agcggttagc tccttcggtc ctccgatcgt tgtcagaagt aagttggccg cagtgttatc 6480
actcatggtt atggcagcac tgcataattc tcttactgtc atgccatccg taagatgctt 6540
ttctgtgact ggtgagtact caaccaagtc attctgagaa tagtgtatgc ggcgaccgag 6600
ttgctcttgc ccggcgtcaa tacgggataa taccgcgcca catagcagaa ctttaaaagt 6660
gctcatcatt ggaaaacgtt cttcggggcg aaaactctca aggatcttac cgctgttgag 6720
atccagttcg atgtaaccca ctcgtgcacc caactgatct tcagcatctt ttactttcac 6780
cagcgtttct gggtgagcaa aaacaggaag gcaaaatgcc gcaaaaaagg gaataagggc 6840
gacacggaaa tgttgaatac tcatactctt cctttttcaa tattattgaa gcatttatca 6900
gggttattgt ctcatgagcg gatacatatt tgaatgtatt tagaaaaata aacaaatagg 6960
ggttccgcgc acatttcccc gaaaagtgcc acctgacgtc 7000